# EUROPEAN PATENT APPLICATION

(11) **EP 4 691 501 A1**
(43) Date of publication of application: **11.02.2026**
(21) Application number: 24836182.6
(22) Date of filing: 14.05.2024
(51) Int. Cl.: A61L 2/20, A61L 2/26, A47L 23/18, A47L 23/20

(54) **SHOE HOLDING DEVICE AND SHOE MANAGEMENT DEVICE INCLUDING SAME**

(30) Priority: 05.07.2023 KR 20230087137
(71) Applicant: LG Electronics Inc., Yeongdeungpo-gu Seoul 07336 (KR)
(72) Inventor: CHUN, Chan Ho, Seoul 08592 (KR); KIM, Ji Hun, Seoul 08592 (KR); PARK, Hye Yong, Seoul 08592 (KR)
(74) Representative: Schornack, Oliver
(86) International application number: PCT/KR2024/006566
(87) International publication number: WO 2025/009733

(57) **Abstract**

A shoe holding device according to an embodiment of the present invention is a holding device configured to hold shoes in a shoe management device having a steam inlet through which steam is supplied to the inside of an inner cabinet and a nozzle through which air is supplied to the inside of the inner cabinet, and comprises a holding base and a holding pipe. The holding base is detachably coupled to the inner cabinet and has a first channel provided therein. A second channel is provided inside the holding pipe. A shoe can be held on the holding pipe, and steam can be intensively and effectively supplied to the shoe via the first channel and the second channel.

## Description

### [Technical Field]

The present invention relates to a shoe holding device and a shoe care device (also referred to as a shoe management device). More specifically, the present inventoin relates to a shoe holding device on which shoes are held such that steam and air can be effectively supplied to the shoes in a shoe care device to which steam and air are supplied.

### [Background Art]

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device, which removes germs and odors by performing a predetermined treatment on the shoes so that a user can comfortably wear the shoes all the times.

Korean Patent Publication No. 10-2023-0026176A (published on February 24, 2023) discloses a shoe care device. In the shoe care device, the shoes are dehumidified and deodorized with a dehumidifying material, and the used dehumidifying material is regenerated such that shoe processing performance can always be maintained appropriately. In addition, the shoe care device may be configured to supply steam to shoes, so that a sterilization effect, inflation processing, or the like of the shoes can be achieved.

There are many types of shoes that need to be managed, such as sneakers, leather shoes, and sandals. The shoes may be made of various materials such as leather, synthetic resin, and vinyl, and even if the shoes are of the same type, the conditions at the time of management may be different.

Therefore, the shoes need to be managed according to the characteristics thereof, and a shoe care device is needed that can provide management suitable for the conditions of various shoes is needed.

In order to effectively remove bacteria or odors from shoes, it is necessary to take intensive care for the shoes, and in that case, it is necessary to remove causes that may damage the shoes.

For example, when supplying steam and air to shoes, it is necessary to intensively and effectively supply steam and air to the shoes, and in this case, it is necessary to ensure that components that may transmit excessive heat to the shoes are separated from the shoes.

### [Disclosure]

### [Technical Problem]

The present disclosure is to provide a shoe holding device that is detachably coupled to an inner cabinet of a shoe care device to which steam and air are supplied and is configured to hold shoes and supply steam at the same time, and a shoe care device including the shoe holding device.

The present disclosure is to provide a shoe holding device including a heating unit provided at the bottom of the inner cabinet of the shoe care device, and configured to separate shoes away from the heating unit when the heating unit is operated and to effectively supply steam to the shoes, and a shoe care device including the shoe holding device.

The present disclosure is to provide a shoe holding device in which the supply of steam and/or air can be prevented from being interrupted by shoes held thereon and the supply of stem and/or air to the shoes may be smoothly achieved, and a shoe care device including the shoe holding device.

The present disclosure is to provide a shoe holding device in which a steam inlet configured to supply steam and a nozzle configured to supply air are individually provided and both the supply of steam and the supply of air to the shoes may be smoothly achieved, and a shoe care device including the shoe holding device.

### [Technical Solution]

A shoe care device described herein may include an inner cabinet, a steam inlet through which steam is supplied into the inner cabinet, and a nozzle through which air is supplied into the inner cabinet.

The shoe holding device described herein is configured to hold a shoe inside the shoe care device.

The shoe holding device includes a holding base and a holding pipe.

The holding base is detachably coupled to the inner cabinet. The holding base is configured to shield the steam inlet. The holding base includes a first channel provided therein to communicate with the steam inlet.

The holding pipe extends upward from the holding base and enters the inside of the shoe. The holding pipe includes a second channel provided therein to communicate with the first channel and a first pipe outlet provided at an end to communicate with the second channel.

The holding pipe may be provided to be inclined upward from the holding base along a first direction which is a horizontal direction.

The holding base may be provided along a second direction, which is a horizontal direction orthogonal to the first direction.

A pair of holding pipes may be provided to be spaced apart from each other in the second direction.

The holding pipe may include a second pipe outlet as a hole provided at a point closer to the first pipe outlet than the holding base and communicating with the second channel.

The holding base may include a base communication hole, which is a hole penetrating the holding base, to allow the first channel to communicate with the inside of the inner cabinet.

The holding pipe may include a third pipe outlet, which is a hole provided at a point closer to the holding base than the first pipe outlet, to allow the second channel to communicate with the inside of the inner cabinet.

The second pipe outlet may be provided in the upper surface of the holding pipe.

The holding base may be coupled to the inner cabinet to be rotatable about a holding rotation shaft. The holding rotation shaft may be parallel to the second direction.

A pair of holding rotation shafts may be provided.

The pair of holding rotation shafts may protrude in the same direction from the holding base.

The holding rotation shafts may be provided at the rear side of the holding base with reference to the first direction.

When the shoe holding device rotates to one side about the holding rotation shaft, the holding pipe may approach a side wall of the inner cabinet and the holding base may expose the steam inlet.

The shoe holding device may further include a holding shelf.

The holding shelf is flat along the horizontal direction to be placed on the bottom of the inner cabinet and is fixed to the holding base.

With reference to the first direction, the holding shelf may have a length greater than the length of the holding pipe.

The shoe holding device may further include a reinforcement rib.

The reinforcement rib is provided to directly interconnect the holding pipe and the holding shelf.

The shoe holding device may further include a nozzle connection part and a channel connection part.

The nozzle connection part protrudes upward from the upper surface of the holding shelf to be connected to the nozzle. The nozzle connection part includes therein a third channel into which air discharged from the nozzle is introduced.

The channel connection part is provided on the upper surface of the holding shelf, and includes a fourth channel provided therein to communicate with the second channel and the third channel.

The fourth channel may be connected to the second channel via the first channel.

The shoe care device may include a heating part.

The heating part may be provided under the bottom of the inner cabinet to which the shoe holding device is coupled, and may be configured to heat air introduced from the inner cabinet.

The shoe care device may include a dehumidifying part.

The dehumidifying part is configured to dehumidify the air introduced from the inner cabinet.

In the shoe care device, air passing through the dehumidifying part may be supplied into the inner cabinet through a nozzle.

The inner cabinet may include a main opening that opens on one side, and the shoe care device may include a door configured to open/close the main opening.

The steam inlet configured to supply steam into the inner cabinet may be provided at the rear bottom of the inner cabinet opposite to the door.

The holding shelf has a plurality of holding shelf holes provided through the holding shelf to allow the air inside the inner cabinet to move toward the heating part.

### [Advantageous Effect]

The shoe holding device according to an embodiment of the present disclosure includes a holding base and a holding pipe. The holding base is detachably coupled to the inner cabinet, and includes therein a first channel. A second channel is provided inside the holding pipe. A shoe can be held on the holding pipe, and steam can be intensively and effectively supplied to the shoe through the first channel and the second channel. The shoe holding device may be detachably coupled to inner cabinet of the shoe care device to be used selectively. By the shoe holding device, holding the shoe and supplying steam can be performed together, and the steam can be intensively and effectively supplied to the shoe.

In the shoe care device according to an embodiment of the present disclosure, a heating part is provided under the bottom of the inner cabinet to which the shoe holding device is coupled, and the heating part may be configured to heat air introduced from the inner cabinet. By operating the heating part, the temperature and humidity of the air used for shoe care can be controlled. At this time, the shoe can be spaced apart from the heating part by being held on the shoe holding device, and the shoe can be prevented from being damaged by the heat from the heating part.

In the shoe care device according to an embodiment of the present disclosure, the holding pipe may include a first pipe outlet and a second pipe outlet. The steam and/or air moving through the second channel of the holding pipe may be supplied into the shoe through the first pipe outlet and the second pipe outlet. Therefore, it is possible to prevent the supply of steam and/or air from being interrupted by the shoe mounted on the shoe holding device, and the supply of steam and/or air to the shoes can be achieved.

The shoe care device according to an embodiment of the present disclosure has a steam inlet configured to supply steam and a nozzle configured to supply air. The shoe holding device has a nozzle connection part and a channel connection part. The air from the nozzle can be supplied to the inside of the shoe after moving to the holding pipe through the nozzle connection part and the channel connection part. Accordingly, the steam supply path and the air supply path can be matched, and both the supply of steam and the supply of air to the shoe can be smoothly achieved.

### [Brief Description of Drawings]

FIG. 1a is a perspective view illustrating a shoe care device according to one embodiment of the present invention.
FIG. 1b is a perspective view of the shoe care device of FIG. 1a, when viewed from another direction, illustrating a state in which a door is opened.
FIG. 2a is a perspective view illustrating a state in which a part of the door and an outer cabinet are removed from the shoe care device of FIG. 1b.
FIG. 2b is a perspective view illustrating a state in which the shoe care device illustrated in FIG. 2a is viewed from another direction.
FIG. 3a is a front view illustrating a state in which a door is removed from the shoe care device illustrated in FIG. 1b.
FIG. 3b is a view illustrating an inner surface of a door with a door seal.
FIG. 4a is a cross-sectional view taken along line A-A' of the shoe care device illustrated in FIG. 3, FIG. 4b is a cross-sectional view taken along line B-B' of the shoe care device illustrated in FIG. 3, and FIG. 4c is a cross-sectional view taken along line C-C' of the shoe care device illustrated in FIG. 3. FIGS. 4a to 4c illustrate a form where a door is included in the shoe care device, and do not illustrate shoes.
FIG. is a perspective view illustrating a state in which the door, the outer cabinet, and the inner cabinet are removed from the shoe care device according to one embodiment of the present invention.
FIG. 6 is a perspective view illustrating a part of a machine room of the shoe care device illustrated in FIG. 5.
FIG. 7a is a view illustrating a steam valve according to one embodiment of the present invention, and illustrating a connection relationship considering the movement of steam. FIG. 7b is an exploded perspective view illustrating the steam valve illustrated in FIG. 7a.
FIG. 8a is a cross-sectional view taken along line D-D' of the shoe care device illustrated in FIG. 3a.
FIG. 8b is a view illustrating a state in which a main shelf is removed from the shoe care device illustrated in FIG. 8a.
FIG. 9 is a cross-sectional view taken along line E-E' of the shoe care device illustrated in FIG. 3a.
FIG. 10a is an exploded perspective view illustrating a drying module in the shoe care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoe care device according to one embodiment of the present invention.
FIG. 12 is an exploded perspective view illustrating the dehumidifying part and the dehumidifying material housing according to one embodiment of the present invention..
FIG. 13 is a bottom perspective view illustrating a module cover according to one embodiment of the present invention.
FIG. 14 is a cross-sectional view illustrating a part of a third module chamber of the module housing in the shoe care device according to the present invention.
FIG. 15 is a view illustrating a portion of the shoe care device of the present disclosure in the state in which shoes are held on the shoe holding device inside the inner cabinet.
FIGS. 16 and 17 are views illustrating the shoe holding device of FIG. 15 as seen from different directions, respectively.
FIG. 18 is a cross-sectional view illustrating the shoe care device of FIG. 15.
FIG. 19A is a cross-sectional view illustrating the shoe care device of FIG. 15, and FIG. 19B is a cross-sectional view illustrating the shoe holding device of FIG. 19A in a rotated state.
FIG. 20 is a view illustrating a partial configuration of the shoe care device according to an embodiment of the present disclosure.
FIG. 21 is a view illustrating a portion of the shoe care device of the present disclosure in the state in which shoes are held on the shoe holding device inside the inner cabinet.
FIGS. 22A and 22B are cross-sectional views each illustrating the shoe care device of FIG. 21.
FIG. 23 is a view illustrating the shoe holding device illustrated in FIG. 21.
FIGS. 24A and 24B are cross-sectional perspective views illustrating the shoe holding device of FIG. 23 when viewed in different directions, respectively.
FIG. 25 is a view illustrating a portion of the shoe care device of the present disclosure in the state in which shoes are held on the shoe holding device inside the inner cabinet.
FIGS. 26A and 26B are cross-sectional views each illustrating the shoe care device of FIG. 25.
FIG. 27 is a view illustrating the shoe holding device illustrated in FIG. 25.
FIGS. 28A to 28C are cross-sectional perspective views illustrating the shoe holding device illustrated in FIG. 27 when viewed in different directions, respectively.

### [Modes for the Invention]

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A care device 1 of the present invention is commenced. The care device 1 may be configured to care the item so that the item becomes clean while the item is accommodated therein or a physical/chemical state of the item accommodated is improved.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

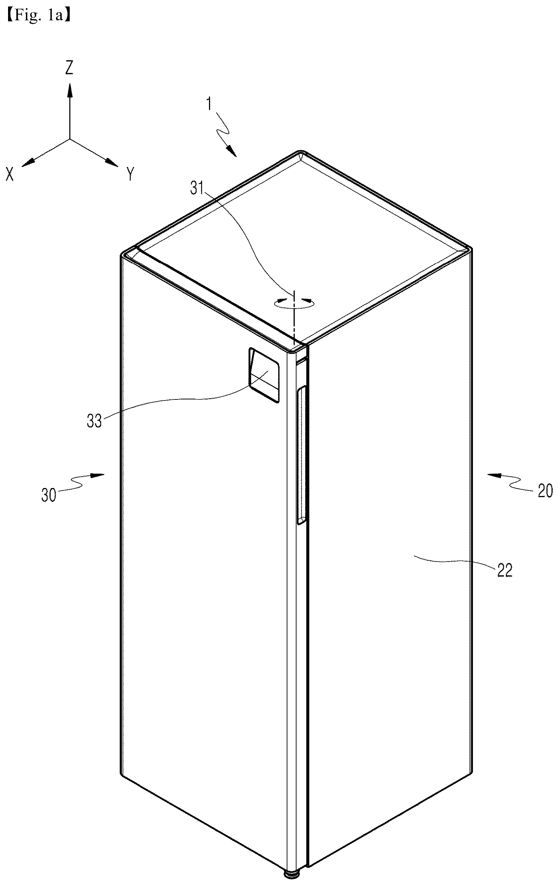

FIG. 1a is a perspective view illustrating a shoe care device 1 according to one embodiment of the present invention.

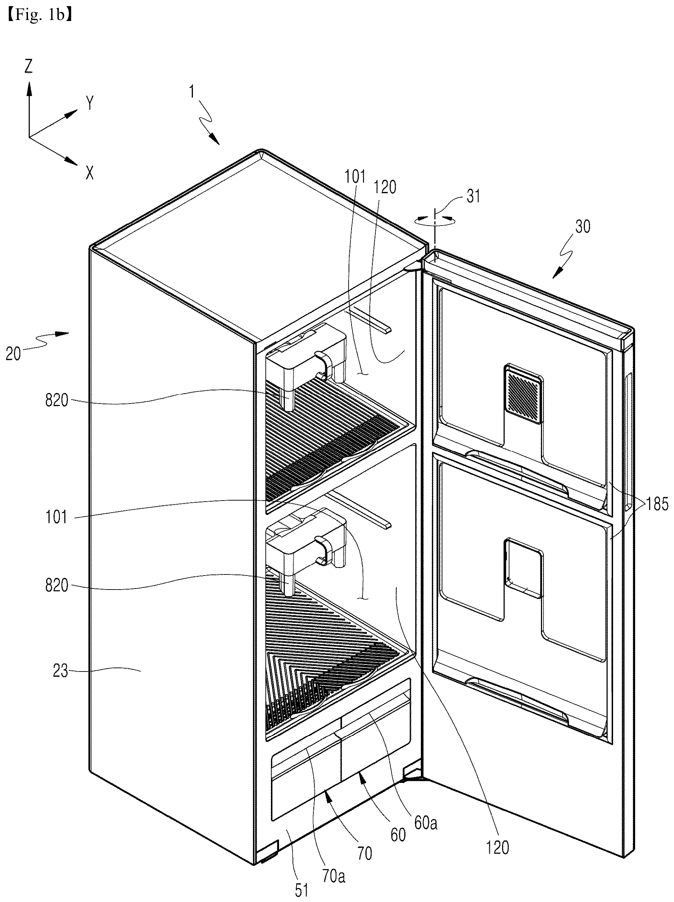

FIG. 1b is a perspective view of the shoe care device of FIG. 1a, when viewed from another direction, illustrating a state in which a door is opened.

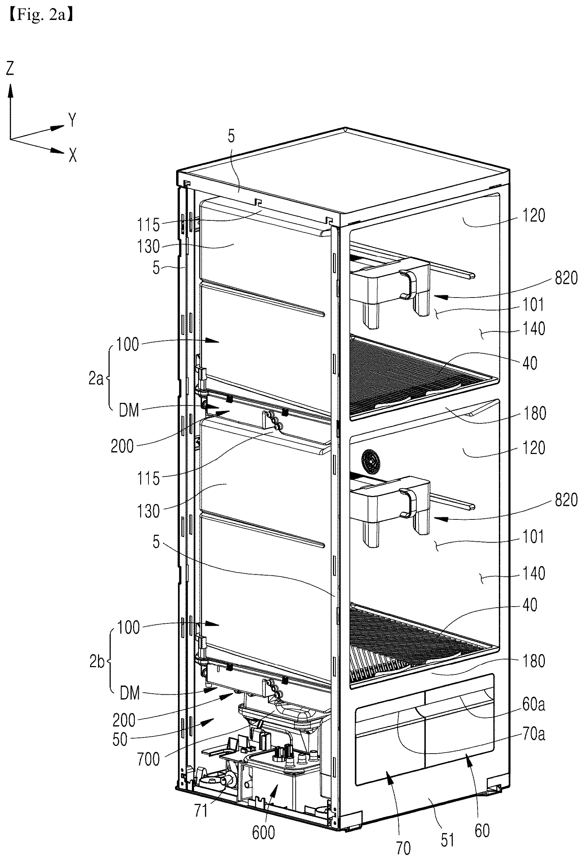

FIG. 2a is a perspective view illustrating a state in which a part of the door and an outer cabinet are removed from the shoe care device of FIG. 1b.

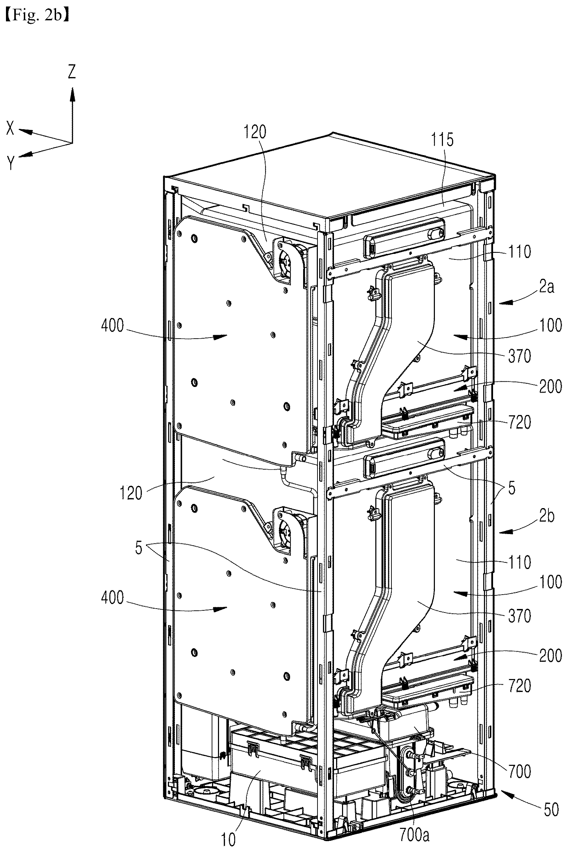

FIG. 2b is a perspective view illustrating a state in which the shoe care device illustrated in FIG. 2a is viewed from another direction.

A shoe care device 1 according to one embodiment of the present invention may include an outer cabinet 20, a door 30, an inner cabinet 100, and a machine room 50. The shoe care device 1 may include a main frame 5.

The outer cabinet 20 and the door 30 may form an overall appearance of the shoe care device 1. The outside of the shoe care device 1 may be formed in a hexahedral shape. That is, while the outer cabinet 20 and the door 30 are coupled to each other and the door 30 is closed, the appearance of the shoe care device 1 may be formed in a hexahedral shape. However, the shoe care device 1 according to one embodiment of the present invention is not limited to such a shape and may have various three-dimensional shapes.

When the door 30 forms the front of the shoe care device 1, the outer cabinet 20 may form an upper side surface, a left-side surface, a right-side surface, a rear surface, and bottom surfaces of the shoe care device 1.

The main frame may form an overall framework of the shoe care device 1. The main frame may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoe care device 1. The outer rear plate 21 may form an entire outer rear surface of the shoe care device 1.

The first outer side plate 22 and the second outer side plate 23 form vertically erected wall surfaces, respectively, and form opposing wall surfaces facing each other.

The first outer side plate 22 and the second outer side plate 23 may be located on both sides with reference to a reference plane RP. The reference plane RP described in an embodiment of the present disclosure refers to a virtual plane parallel to the first direction (X direction) which is a horizontal direction and extends along a vertical direction. The reference plane RP described in an embodiment of the present disclosure may refer to a virtual vertical plane that crosses the center of the shoe care device 1 in the front-rear direction.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoe care device 1, the outer cabinet 20 may form a wall separating the machine room 50 from the outside.

The door 30 is configured to open and close the inside of the shoe care device 1. The door 30 may form any one surface of the shoe care device 1. The door 30 may form a left side or a right side of the shoe care device 1, or may form a front side of the shoe care device 1.

In the shoe care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoe care device 1, the door 30 may be configured to be rotatable bidirectionally around a rotation axis 31 in the vertical direction.

In one embodiment, the shoe care device 1 may include one door 30. In another embodiment, the shoe care device 1 may include two or more doors.

When two doors are provided in the shoe care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoe care device 1 to the front.

Hereinafter, except for a particularly limited case, it will be described that the door 30 is formed in a front side of the shoe care device 1. That is, a surface on which the door 30 is formed in the shoe care device 1 is described as a front surface of the shoe care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoe care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 11may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100.

However, the shoe care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoe care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoe care device 1 may be accommodated, and, in this case, the components that are accommodated in the machine room 50 may be fixed to a main frame or to the inner cabinet 100 or the outer cabinet 20.

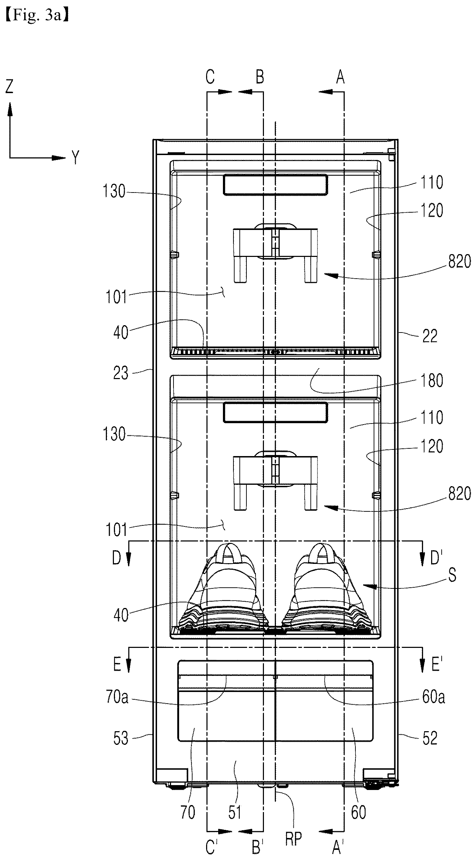

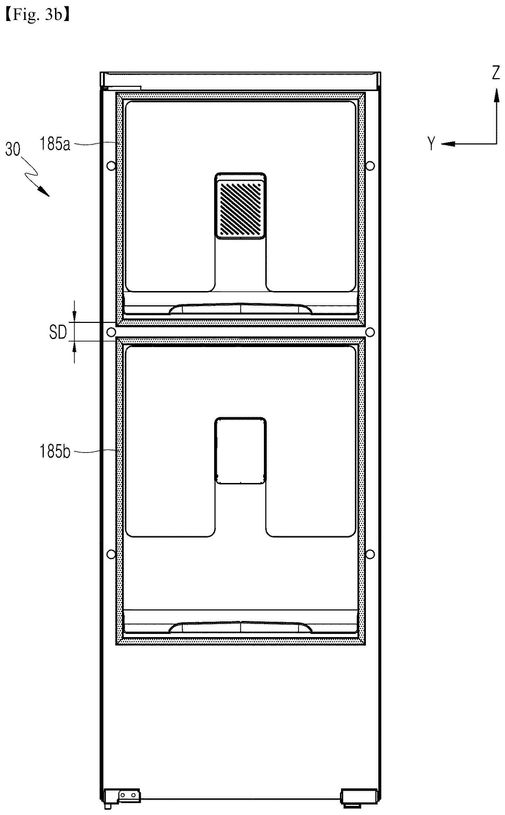

FIG. 3a is a front view illustrating a state in which a door is removed from the shoe care device illustrated in FIG. 1b. FIG. 3a illustrates shoes accommodated in an inner cabinet together. FIG. 3b is a view illustrating an inner surface of a door 30 with door seals 185a and 185b.

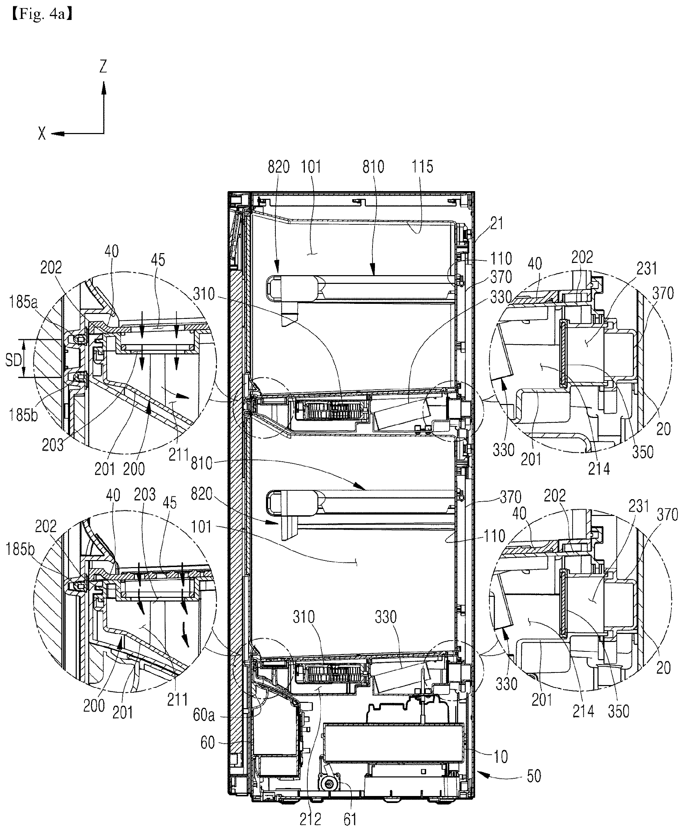

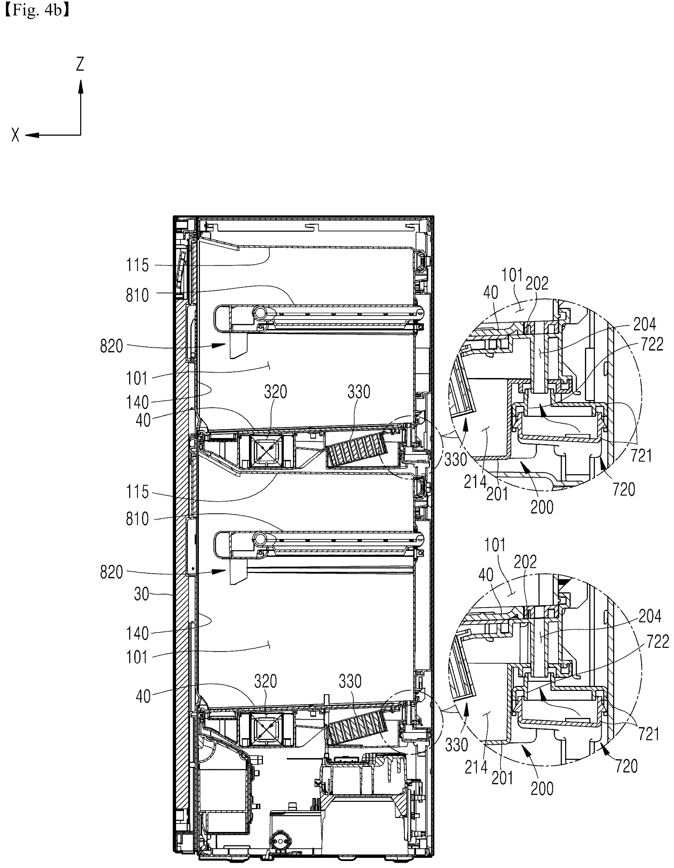

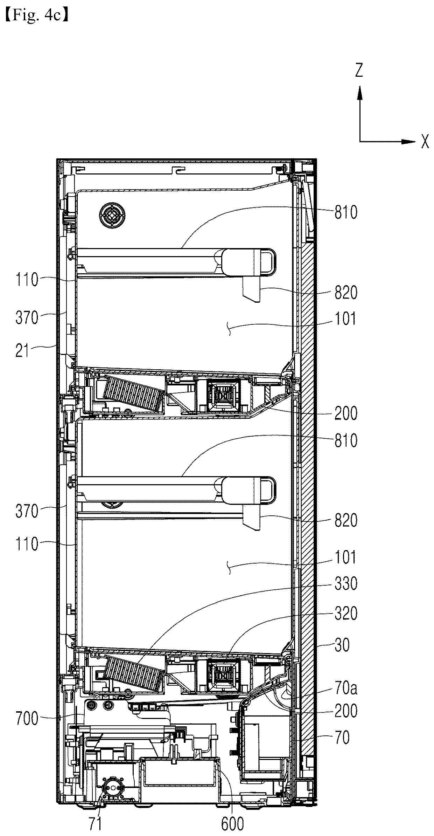

FIG. 4a is a cross-sectional view taken along line A-A' of the shoe care device illustrated in FIG. 3a, FIG. 4b is a cross-sectional view taken along line B-B' of the shoe care device illustrated in FIG. 3a, and FIG. 4c is a cross-sectional view taken along line C-C' of the shoe care device illustrated in FIG. 3a. FIGS. 4a to 4c illustrate a form where a door is included in the shoe care device, and do not illustrate shoes.

The shoe care device 1 according to one embodiment of the present invention includes management devices 2a and 2b. In the shoe care device 1, a plurality of management devices 2a and 2b may be provided. In one embodiment, the shoe care device 1 may include a first management device 2a and a second management device 2b. That is, the shoe care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoe care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The shoe care device 1 according to the embodiment of the present invention may simultaneously care two or more different shoes (a first shoe, a second shoe, etc.). Any one of the first shoe and the second shoe may be accommodated and cared in the inner cabinet 100 of the first management device 2a, and the other one may be accommodated and cared in the inner cabinet 100 of the second management device 2b. The firs shoe and the second shoe may be made of different materials, or configured to be in different states or to have different features. In this case, the first shoe and the second shoe need to be care in different schemes and/or different conditions.

When it is preferable to care the first shoe by using the steam, it may be preferable to care the second shoe without providing the steam. In this case, in the shoe care device 1, the steam may be supplied to the accommodation space 101 of the inner cabinet 100 of the first management device 2a, which accommodates the first shoe, and not supplied to the accommodation space 101 of the inner cabinet 100 of the second management device 2b, which accommodates the second shoe.

When it is preferable to care the first shoe by using a relatively large amount of steam, it may be preferable to care the second shoe by using a relatively small amount of steam. In this case, in the shoe care device 1, the relatively large amount of steam may be supplied to the accommodation space 101 of the inner cabinet 100 of the first management device 2a, which accommodates the first shoe, and the relatively small amount of steam may be supplied to the accommodation space 101 of the inner cabinet 100 of the second management device 2b, which accommodates the second shoe.

In an embodiment, when the first shoe is a sports shoe, the second shoe may be a shoe (a leather shoe).

In the embodiment of the present invention, the door 30 may close the accommodation space 101 of the first management device 2a and the accommodation space 101 of the second management device 2b not to be in communication with each other, and as a result, the movement of the steam may be interrupted between the inner cabinet 100 of the first management device 2a and the inner cabinet 100 of the second management device 2b. As a result, in the shoe care device 1 according to the embodiment of the present invention, a shoe (e.g., a first shoe) of which processing by the steam is required and a shoe (e.g., a second shoe) of which processing by the steam should be prevented can be simultaneously effectively cared.

In order to stably seal the main opening 140 of the inner cabinet 100, the shoe care device 1 may be configured to include a door seal 185. The door seal 185may be made of an elastically transformable material such as rubber. The door seal 18has a rectangular shape as a whole to be coupled to the inner surface of the door 30. When the door 30 is closed, the door seal 18may be close attached along a border of the main opening 140 of the inner cabinet 100. The door seal 18may be closely attached to a front frame 180 of the inner cabinet 100. In the shoe care device 1, two door seals 18may be provided, and one door seal 185a (first door seal) among the door seals 18may be closely attached to the border of the main opening 140 of the inner cabinet 100 of the first management device 2a, and the other one door seal 185b (second seal) may be closely attached to the border of the main opening 140 of the inner cabinet 100 of the second management device 2b.

In the shoe care device 1 according to an embodiment of the present invention, the main opening 140 of the inner cabinet 100 of the first management device 2a and the main opening 140 of the inner cabinet 100 of the second management device 2b may be simultaneously opened/closed. As a result, a plurality of inner cabinets 100 may be easily opened/closed.

Further, according to the embodiment of the present invention, the plurality of inner cabinets 100 is opened/closed by one door 30, and as a result, generation of a gap depending on provision of a plurality of doors (a gap between the doors), a step between the doors, interference, etc. may be fundamentally prevented and since an entire front surface of the shoe care device 1 may be formed by one door 30, a clean and beautiful external appearance may be formed.

In the embodiment of the present invention, the first door seal 185a and the second door seal 185b are jointly coupled to the inner surface of one door 30, and as a result, a spacing distance SD between the first door seal 185a and the second door seal 185b may be formed to be comparatively narrow, and as a result, there is an effect that the accommodation space 101 of the first management device 2a and the accommodation space 101 of the second management device 2b may be formed to be wider.(see Fig. 4a)

Unlike this, when each of the door (first door) of opening/closing the accommodation space 101 of the first management device 2a and the door (second door) of opening/closing the accommodation space 101 of the second management device 2b is individually provided, it is difficult to couple the door seal to a border end of each door in manufacturing, the SD between the first door seal and the second door seal is relatively widened, and consequently, the volume of each accommodation space is narrowed.

In the embodiment of the present invention, the first door seal 185a and the second door seal 185b are jointly coupled to the inner surface of one door 30, and as a result, each of the accommodation space 101 of the first management device 2a and the accommodation space 101 of the second management device 2b may be more effectively sealed.

When the air in the accommodation space 101 of the first management device 2a intends to move downward, the accommodation space is first primarily interrupted by the first door seal 185a, and secondarily interrupted by the second door seal 185b. Further, when the air in the accommodation space 101 of the first management device 2a intends to move upward, the accommodation space is first primarily interrupted by the second door seal 185b, and secondarily interrupted by the first door seal 185a.

The management devices 2a and 2b may be configured to include a connection path F10. The management devices 2a and 2b may be configured to include a blowing part 310 and a dehumidifying part 330. The management devices 2a and 2b may include an outlet 203 and a nozzle 820.

The management devices 2a and 2b may include the module housing 200 forming a connection path F10. The module housing 200 forms all or part of the connection path F10

The management devices 2a and 2b may include a regeneration path F20. The management devices 2a and 2b may include a heating part 320.

The management devices 2a and 2b may include a conversion flow path F10a.

The management devices 2a and 2b may include a damper 350.

The shoe care device 1 may include a steam generator 700 and a steam valve 710.

The shoe care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoe care device 1 may be provided, and various flow paths constituting the shoe care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoe care device 1 moves.

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoe care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoe care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 4penetrating in the vertical direction may be formed in the main shelf 40 so as to move the air.

In the shoe care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoe care device 1 according to one embodiment of the present invention.

The inner cabinet 100, the module housing 200, and the machine room 50 may be continuously arranged from the upper side to the lower side. When the shoe care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoe care device 1.

In the shoe care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoe care device 1 inside. The water supply tank 60 may be configured to store water supplied into the steam generator 700 inside.

In order to supply water from the water supply tank 60 into the shoe care device 1, a water pump 61 may be connected to the water supply tank 60. The water supply tank 60 for moving water and the first water pump 61 may be connected by a pipe, a hose, etc.

The drain tank 70 may be configured to store water discharged from the shoe care device 1 inside. The drain tank 70 may store water condensed inside the shoe care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoe care device 1.

In order to control the controller 10, the shoe care device 1 may be provided with a storage medium in which an application program is stored, and the controller 10 may be configured to control the shoe care device 1 by driving an application program according to information input to the shoe care device 1 and information output from the shoe care device 1.

The controller 10 may control the first management device 2a and the second management device 2b constituting the shoe care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoe care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoe care device 1, the door 30, the water supply tank 60, and the drain tank 70 are formed on the same side, and when the door 30 is opened, the water supply tank 60 and the drain tank 70 may be exposed and separated from the shoe care device 1.

In the arrangement explained above, even if left and right sides and a rear side of the shoe care device 1 are blocked by other goods or structures, the door 30 may be opened on a front side of the shoe care device 1, and the water supply tank 60 and the drain tank 70 can be separated from or coupled again to the shoe care device 1.

A control panel 33 for controlling the shoe care device 1 is provided on an outer side of the door 30. The control panel 33 may be formed of a touch screen. A controller (controller 10) is provided in the inner space of the door 30 to control each component of the shoe care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoe care device 1 according to one embodiment of the present invention may be further provided with a dedicated door of the machine room 50 to open and close the machine room 50 separately from the door 30.

FIG. is a perspective view illustrating a state in which the door 30, the outer cabinet 20, and the inner cabinet 100 are removed from the shoe care device 1 according to one embodiment of the present invention.

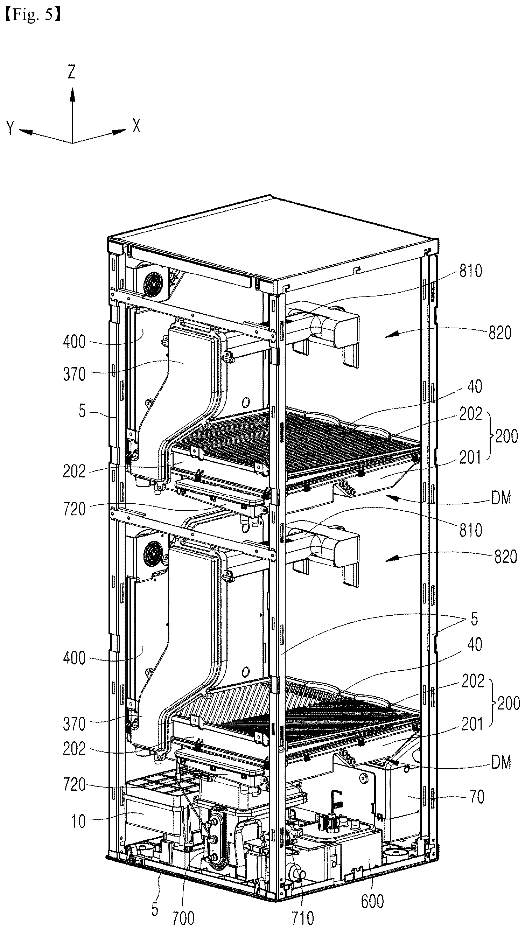

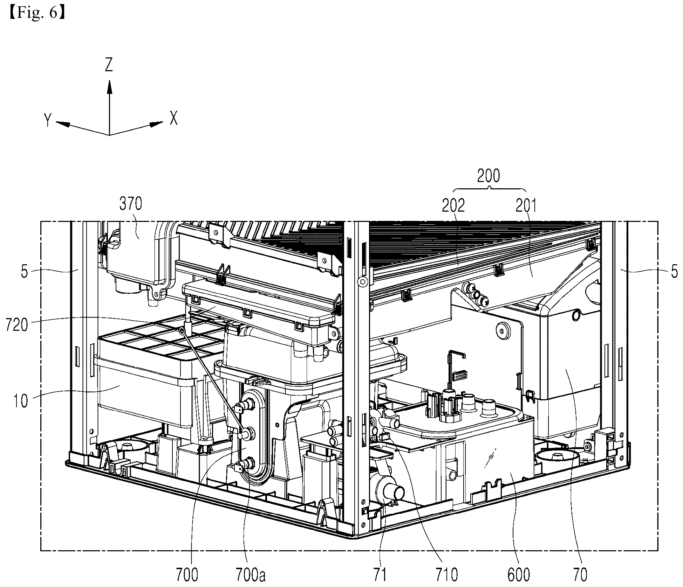

FIG. 6 is a perspective view illustrating a machine room part of the shoe care device 1 illustrated in FIG. 5.

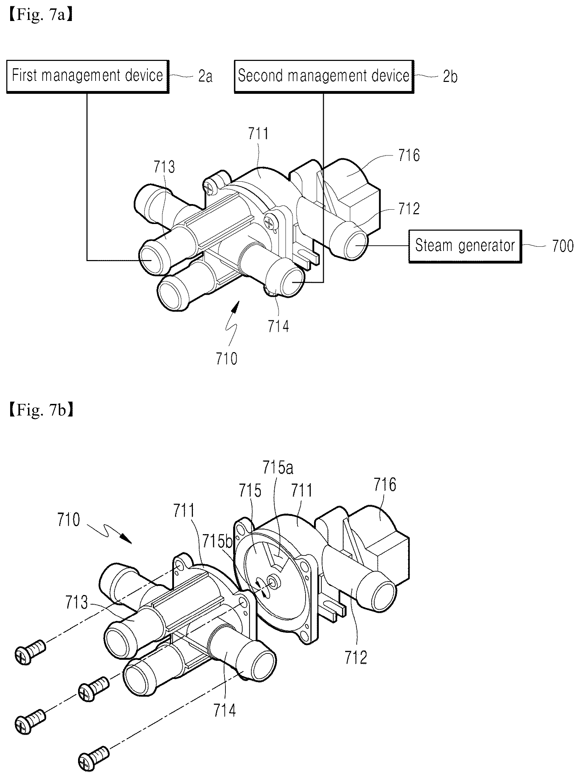

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoe care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoe care device 1 according to one embodiment may be provided with one steam generator 700, and the shoe care device 1 according to another embodiment may be provided with two or more steam generators 700.

When the shoe care device 1 is provided with one steam generator 700, the steam generator 700 may be configured to supply steam into the inner cabinet 100 of the first management device 2a and/or into the inner cabinet 100 of the second management device 2b.

When the shoe care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoe care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

An external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoe care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 71is provided inside the steam valve 710 (inside the valve housing 711) and is configured to open and close a flow path inside the steam valve 710. The valve disk 71may be configured to selectively open and close a flow path of the first valve outlet 713 and a flow path of the second valve outlet 714.

The valve disk 71is disposed between the valve inlet 712 and the first valve outlet 713, or between the valve inlet 712 and the second valve outlet 714, inside the valve housing 711. The valve disk 71allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 71may be formed in a circular plate shape and may include a valve hole 715a. The valve hole 715a is a hole penetrating the valve disk 715. The valve disk 71may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 71to rotate the valve disk 715.

The controller 10 may control the steam valve 710 by controlling an operation of the valve motor 716.

The valve disk 71rotates by the operation of the valve motor 716, and the valve disk 71opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoe care device 1 according to one embodiment of the present invention, the valve disk 71may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoe care device 1, the steam may be sufficiently and stably supplied to the accommodation spaces 101 of each of the two inner cabinets 100.

As such, in the embodiment of the present invention, the steam generated in one steam generator 700 is selectively supplied to any one of the respective accommodation spaces 101 of two inner cabinets 100, and as a result, the steam may be stably supplied to the accommodation space 101 of the first management device 2a or the accommodation space 101 of the second management device 2b without pressure drop.

When the accommodation space 101 of the first management device 2a and the accommodation space 101 of the second management device 2b are vertically placed, if the steam is configured to be supplied to both the accommodation space 101 of the first management device 2a and the accommodation space 101 of the second management device 2b, most steam may be supplied only to any one accommodation space 101 (e.g., the accommodation space 101 of the second management device 2b positioned at the lower side) and the steam is not almost supplied to the other one accommodation space 101 (e.g., the accommodation space 101 of the first management device 2a) or only a small amount of steam may be supplied, but the steam may be supplied in the embodiment of the present invention to prevent such a problem.

In one embodiment of the present invention, the controller 10 may control the stream valve 710 such that when the heating part 320 of the first management device 2a is turned off (when a heater 321 of the heating part 320 is turned off), the valve disk 71closes or opens the first valve outlet 713, and when the heating part 320 of the first management device 2a is turned on (when the heater 321 of the heating part 320 is turned on), the valve disk 71closes the first valve outlet 713.

In addition, the controller 10 may control the stream valve 170 such that when the heating part 320 of the second management device 2b is turned off (when the heater 321 of the heating part 320 is turned off), the valve disk 71closes or opens the second valve outlet 714, and when the heating part 320 of the second management device 2b is turned on (when the heater 321 of the heating part 320 is turned on), the valve disk 71closes the second valve outlet 714.

In the shoe care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoe care device 1.

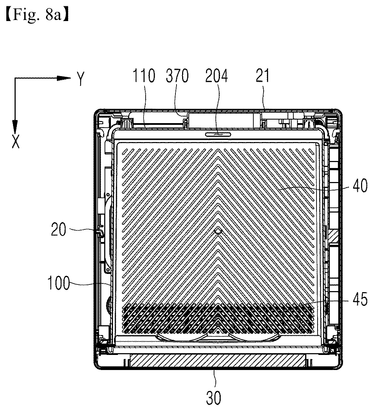

FIG. 8a is a cross-sectional view taken along line D-D' of the shoe care device 1 illustrated in FIG. 3a.

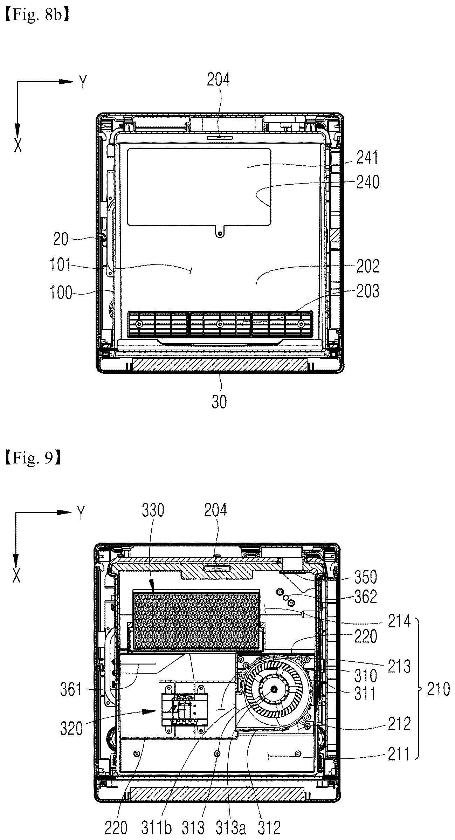

FIG. 8b is a view illustrating a state in which the main shelf 40 is removed from the shoe care device 1 illustrated in FIG. 8a.

FIG. 9 is a cross-sectional view taken along line E-E' of the shoe care device 1illustrated in FIG. 3a.

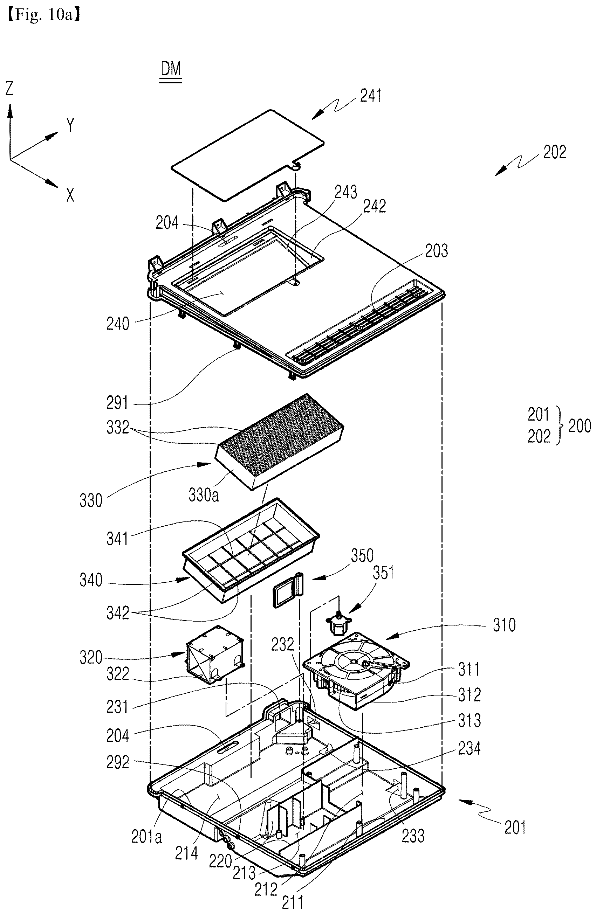

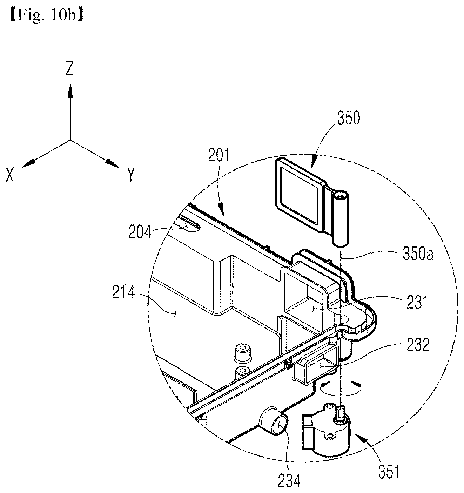

FIG. 10a is an exploded perspective view illustrating a drying module in the shoe care device 1 according to one embodiment of the present invention. FIG. 10b is an exploded perspective view illustrating a partial configuration of the drying module at a part to which the damper 350 is coupled.

In the shoe care device 1 according to one embodiment of the present invention, the dehumidifying part 330 may be used as a means that dehumidifies air.

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (Å) are regularly arranged, and may function as dehumidification by adsorbing the moisture in the air.

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A housing 311 of a blowing fan 313 may be provided inside the blowing part 310. The blowing fan 313 may be configured to rotate around a rotation axis 313a parallel to the third direction (Z direction) inside the housing 311 of the blowing part 310, and thus air is blown through an outlet 311b of the housing 311. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of p the ventilation art 310 (a rotation of the blowing fan 313), and the air inside the connection path F10 may be ventilated.

When the blowing fan 313 operates, air flows into the housing 311 from the blowing part 310 through an opening at the lower side of the housing 311, and flows out through the outlet 311b provided at an end of the blowing duct 313.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoe care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed.

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoe care device 1 according to one embodiment may be provided with two sets.

Such a set may be provided in each of the first management device 2a and the second management device 2b.

Such a set may form a drying module DM in the shoe care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoe care device 1 according to one embodiment of the present invention, a plurality of drying modules DM may be provided.

In the shoe care device 1 according to one embodiment of the present invention, a pair of drying modules DM may be provided. When the shoe care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoe care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoe care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoe care device 1 according to one embodiment of the present invention may be formed in a structure in which the dehumidifying part 330 may be detachable from the module housing 200. The structure of the shoe care device provides an advantageous advantage in maintaining and managing the dehumidifying part 330 and the shoe care device 1 on the whole.

On the other hand, the dehumidifying part 330 may be repeatedly used by regeneration, but with the repeated use, the dehumidifying part 330 needs to be replaced.

Considering these descriptions, the shoe care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoe care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoe care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoe care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoe care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user.

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoe care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoe care device 1, such moisture may reproduce bacteria or cause odors. This is why there is need for countermeasures to solve the problems, and the shoe care device 1 according to one embodiment of the present invention can effectively prevent water from leaking in consideration of such problems.

In the shoe care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoe care device 1 with excellent regeneration efficiency.

In the shoe care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoe care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoe care device 1 according to one embodiment of the present invention, steam generated by the steam generator 700 is supplied to the accommodation space 101 of the inner cabinet 100, and to this end, the shoe care device 1 includes a steam inlet 204.

The steam inlet 204 forms an inlet through which steam is supplied to the accommodation space 101 of the inner cabinet 100.

In the shoe care device 1 according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam generator 700 and the steam valve 710 are disposed in a lower part, the distance from the steam generator 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam generator 700 to the steam inlet 204

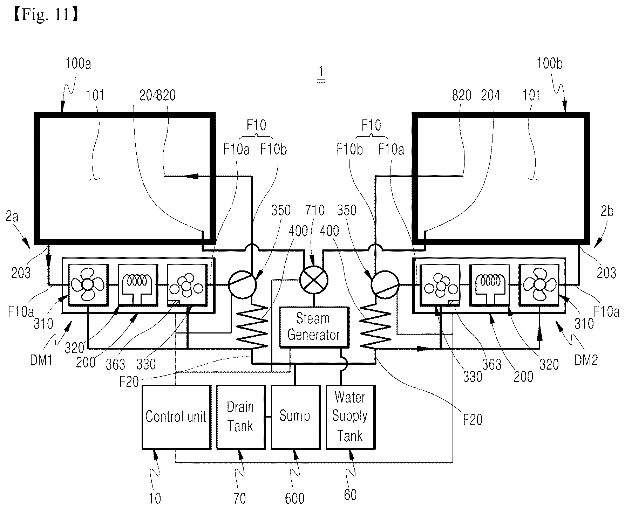

FIG. 11 is a diagram illustrating a connection relationship between components and a flow of fluid in the shoe care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoe care device 1.

In the nozzle 820, a hole through which air is discharged is formed in the accommodation space 101 of the inner cabinet 100, and the nozzle 820 may form a last part of the connection path F10.

In the shoe care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the blowing part 310, the heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 (the dehumidifying material 331) are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

The dry air duct 370 may be fixedly coupled to an outer wall surface of the inner cabinet 100, and the nozzle duct 810 may be provided inside the inner cabinet 100.

The nozzle duct 810 may be hinged to the inner cabinet 100, and the nozzle 820 may be hinged to the nozzle duct 810. At this time, the hinge axis of the nozzle duct 810 and the hinge axis of the nozzle 820 are each parallel to the horizontal direction (second direction (Y direction)). When the nozzle duct 810 rotates about its hinge axis relative to the inner cabinet 100, the position of the nozzle 820 may move in the vertical direction.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration path F20 forms a passage through which air and/or condensed water inside the shoe care device moves.

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoe care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoe care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoe care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoe care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path F10a and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoe care device 1 according to one embodiment of the present invention, the first management device 2a and the second management device 2b individually include the inner cabinet 100, the connection path F10, the blowing part 310, and the dehumidifying part 330. In addition, the shoe care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoe care device 1, and the shoe care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoe care device 1 can be achieved.

In the shoe care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoe care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 71closes or opens the first valve outlet 713, when the heating part 320 of the first management device 2a is turned on, the valve disk 71closes the first valve outlet 713, when the heating part 320 of the second management device 2b is turned off, the valve disk 71closes or opens the second valve outlet 714, and when the heating part 320 of the second management device 2b is turned on, the valve disk 71closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoe care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoe care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoe care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoe care device 1 operates to refresh the shoes.

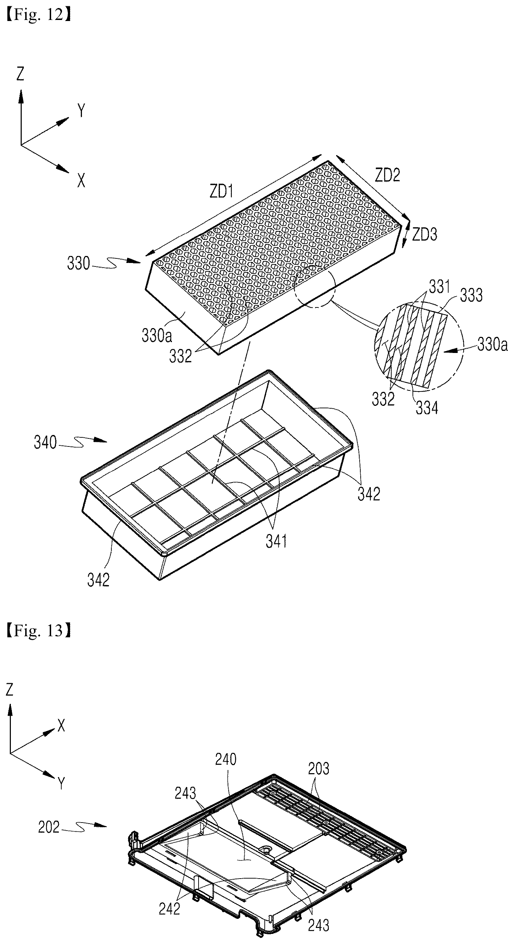

FIG. 12 is an exploded perspective view illustrating the dehumidifying part 330 and the dehumidifying material housing 340 according to one embodiment of the present invention.

FIG. 13 is a bottom perspective view illustrating a module cover 202 according to one embodiment of the present invention.

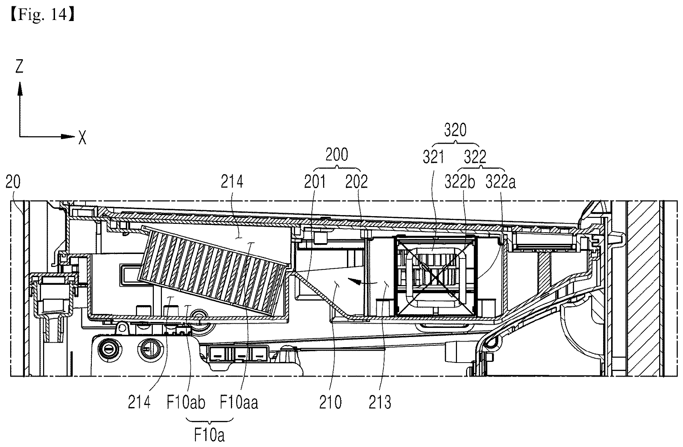

FIG. 14 is a cross-sectional view illustrating a part of a third module chamber 214 of the module housing 200 in the shoe care device 1 according to the present invention..

The dehumidifying part 330 according to one embodiment of the present invention may be configured to have a predetermined thickness and length. In one embodiment, as described above, the dehumidifying part 330 may be configured to have a substantially hexahedral shape.

Accordingly, the dehumidifying part 330 may have a predetermined length, width, and thickness.

Each of a length ZD1 and a width ZD2 of the dehumidifying part 330 may be formed longer than a thickness ZD3 of the dehumidifying part 330. In one embodiment, the length ZD1 and the width ZD2 of the dehumidifying part 330 be made more than twice the thickness ZD3 of the dehumidifying part 330. In addition, the length ZD1 of the dehumidifying part 330 may be formed longer than the width ZD2 of the dehumidifying part 330.

The dehumidifying part 330 includes an upper surface 333 and a lower surface 334 facing each other. Here, the upper surface 333 of the dehumidifying part 330 and the lower surface 334 of the dehumidifying part 330 are opposite surfaces facing each other in the thickness direction of the dehumidifying part 330.

As described above, the dehumidifying part 330 is provided with the plurality of dehumidification through holes 332. The dehumidifying through hole 332 may be configured to penetrate the dehumidifying part 330 in a direction in which the upper surface 333 and the lower surface 334 of the dehumidifying part 330 are connected to each other.

In the shoe care device 1 according to one embodiment of the present invention, the dehumidifying part 330 may be accommodated inside the module housing 200 while being accommodated in the dehumidifying material housing 340.

The dehumidifying material housing 340 is formed in the form of a container capable of accommodating the dehumidifying part 330. In a state where the dehumidifying part 330 is accommodated in the dehumidifying material housing 340, an edge wall of the dehumidifying material housing 340 may be in close contact with the dehumidifying part 330. Accordingly, the dehumidifying part 330 can be prevented from being separated inside the dehumidifying material housing 340.

The dehumidifying material housing 340 has an upper side opened and a lower side opened. However, the lower side of the dehumidifying material housing 340 is provided with a support 341 that supports the dehumidifying part 330 such that the dehumidifying part 330 does not deviate downwards. The supports 341 may be disposed to cross each other in the form of a grid or a net. The gap (opening) between the supports 341 is formed sufficiently larger than the dehumidifying through hole 332 so as not to interfere with the flow of the air passing through the dehumidifying part 330.

In the shoe care device 1 according to one embodiment of the present invention, when the dehumidifying part 330 is disposed inside the module housing 200, the dehumidifying part 330 may be disposed so that the upper surface 333 and the lower surface 334 are inclined without being parallel to the horizontal direction.

The dehumidifying part 330 may be disposed in the third module chamber 214 in a form inclined downwardly toward the second module chamber 213. That is, the upper surface 333 and the lower surface 334 of the dehumidifying part 330 may be disposed to be inclined downwardly toward the second module chamber 213.

Since the dehumidifying part 330 is inclined downwardly toward the second module chamber 213 in the third module chamber 214, the air before penetrating the dehumidifying part 330 is disposed in an upper space of the dehumidifying part 330 in the third module chamber 214, and the air after penetrating the dehumidifying part 330 is disposed in a lower space of the dehumidifying part 330 in the third module chamber 214. Here, the upper space of the dehumidifying part 330 in the third module chamber 214 is defined as 'a first flow path F10aa', and the lower space of the dehumidifying part 330 in the third module chamber 214 is defined as 'a second flow path F10ab.

In addition, since the dehumidifying part 330 is disposed to be inclined in the third module chamber 214, the dehumidifying through hole 332 is also disposed to be inclined.

A cover partition wall 242 may be formed in the module cover 202.

The cover partition wall 242 is formed in a plate shape extending downwards from the edge of the dehumidifying material exit 240. The cover partition wall 242 may be formed in a substantially triangular plate shape.

The cover partition wall 242 may be configured such that a lower edge thereof is inclined downwards from the third module chamber 214 to the second module chamber 213. A pair of cover partition walls 242 may be provided to be spaced apart from each other in the left-right direction. The distance between the pair of cover partition walls 242 may be configured to correspond to the length of the dehumidifying part 330.

A lower locking portion 243 may be formed in a lower edge of the cover partition wall 242 and a front edge of the cover partition wall 242 in the first direction (X direction), and an upper locking portion 342 may be formed in an upper edge of the dehumidifying material housing 340 to get settled and locked in an upper side of the lower locking portion 243. Accordingly, when the dehumidifying material housing 340 is settled in the cover partition wall 242 in a state where the dehumidifying part 330 is accommodated in the dehumidifying material housing 340, the upper locking portion 342 of the dehumidifying material housing 340 is settled and assembled in an upper side of the lower locking portion 243 of the cover partition wall 242.

In addition, in this case, the cover partition wall 242 may block direct communication between the first flow path F10aa and the second flow path F10ab while the lower edge thereof is in close contact with the upper edge of the dehumidifying part 330.

By providing the cover partition wall 242 in the module cover 202, the air in the first flow path F10aa may pass through the dehumidifying part 330 over the entire area of the dehumidifying part 330 and move to the second flow path F10ab. In addition, a plurality of dehumidifying through holes 332 penetrating the dehumidifying part 330 in the thickness direction may be formed in the dehumidifying part 330, thereby increasing the contact area between the air passing through the third module chamber 214 and the dehumidifying material 331.

In one embodiment of the present invention, the dehumidifying part 330 is not disposed parallel to the horizontal direction and is inclined downwardly toward the second module chamber 213. Comparing this with the case where the dehumidifying part 330 is disposed horizontally, the size of the upper surface of the dehumidifying part 330 may be formed larger, and the entire volume of the dehumidifying part 330 may be increased. Accordingly, the amount of dehumidification of air by the dehumidifying part 330 may be increased.

As described above, as the dehumidifying part 330 is disposed to be inclined, the direction from the first flow path F10aa to the second flow path F10ab through the dehumidifying part 330 is configured not to be vertical but to be inclined. A rapid change in the direction of air in a path through which the air moves to the second module chamber 213, the first flow path F10aa, and the second flow path F10ab can be reduced to achieve a smooth movement of air.

Accordingly, the natural movement of air moving through the dehumidifying part 330 can be achieved, and an unnecessary flow path resistance can be minimized in the third module chamber 214.

In addition, while the air of the second module chamber 213 moves from the first flow path F10aa to the second flow path F10ab, moisture (small droplets or condensed water) may enter or occur in the third module chamber 214. The bottom surface of the dehumidifying part 330 may be naturally separated from the bottom of the third module chamber 214, and the air inside the third module chamber 214 may move downwards through the dehumidifying part 330 from the upper side of the third module chamber 214. Accordingly, the small droplets or the condensed water can move along the bottom surface of the third module chamber 214 without remaining or seeping into the dehumidifying part 330, and can be easily discharged toward the condenser 400.

In one embodiment, the dehumidifying part 330 may have a constant cross-section along the second direction (Y direction).

In another embodiment, the dehumidifying part 330 may be formed in a form where the thickness thereof is deformed along the second direction (Y direction).

In the shoe care device 1 according to one embodiment of the present invention, the first module chamber 212 and the second module chamber 213 are formed in front of the third module chamber 214 with respect to the first direction (X direction). That is, when the longitudinal direction of the dehumidifying part 330 is formed along the second direction (Y direction), the first module chamber 212 or the second module chamber 213 does not interfere with securing the length of the third module chamber 214, and the blowing part 310 or the heating part 320 does not interfere with securing the length of the dehumidifying part 330. Accordingly, a total length ZD1 of the dehumidifying part 330 may be formed longer than the length of each of the blowing part 310 and the heating part 320 with respect to the second direction (Y direction).

Accordingly, the length of the dehumidifying part 330 can be secured sufficiently long, the entire volume of the dehumidifying part 330 can be formed relatively large, and the dehumidifying amount per unit time of the dehumidifying part 330 can be improved.

In the shoe care device 1 according to one embodiment of the present invention, the length ZD1 of the dehumidifying part 330 may be longer than 1/2 of the length of each of the inner cabinet 100 and the module housing 200 with respect to the second direction (Y direction).

The dehumidifying part 330 may be spaced apart from the bottom of the third module chamber 214 so that the air inside the third module chamber 214 moves downwards through the dehumidifying part 330 from the upper side of the third module chamber 214. Since the dehumidifying part 330 is spaced apart from the bottom of the third module chamber 214, the air inside the third module chamber 214 can smoothly pass through the dehumidifying part 330, and the condensed water generated by penetrating the dehumidifying part 330 moves along the bottom surface of the third module chamber 214 and can be discharged outside the module housing 200.

In the shoe care device 1 according to one embodiment of the present invention, the dehumidifying part 330 provided in the third module chamber 214 may be disposed to be inclined. The dehumidifying part 330 may be disposed to be inclined downwardly toward the second module chamber 213. Accordingly, when the air moves from the second module chamber 213 to the third module chamber 214, the air can easily pass through the dehumidifying part 330. In addition, compared to the case where the dehumidifying part 330 is horizontally arranged, the area or volume of the dehumidifying part 330 can be further expanded, and the dehumidifying amount per unit time of the dehumidifying part 330 can be improved.

The dehumidifying part 330 according to an embodiment of the present disclosure is obtained by coating pulp-based fabric with a zeolite component (containing silicon dioxide, titanium dioxide, and the like as main components). Here, the pulp-based fabric has a honeycomb structure, and the diameter of each channel (dehumidifying through-hole 332) of the honeycomb structure may be about 2 mm, and the cross section of the channel may be triangular, square, hexagonal, or the like.

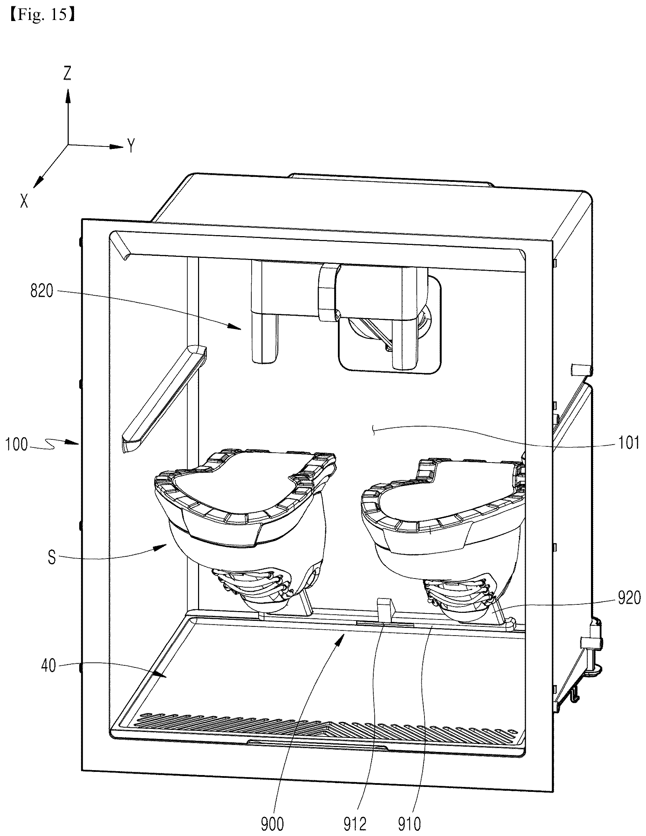

FIG. 15 is a view illustrating a portion of the shoe care device 1 of the present disclosure in the state in which shoes S are held on the shoe holding device 900 inside the inner cabinet 100.

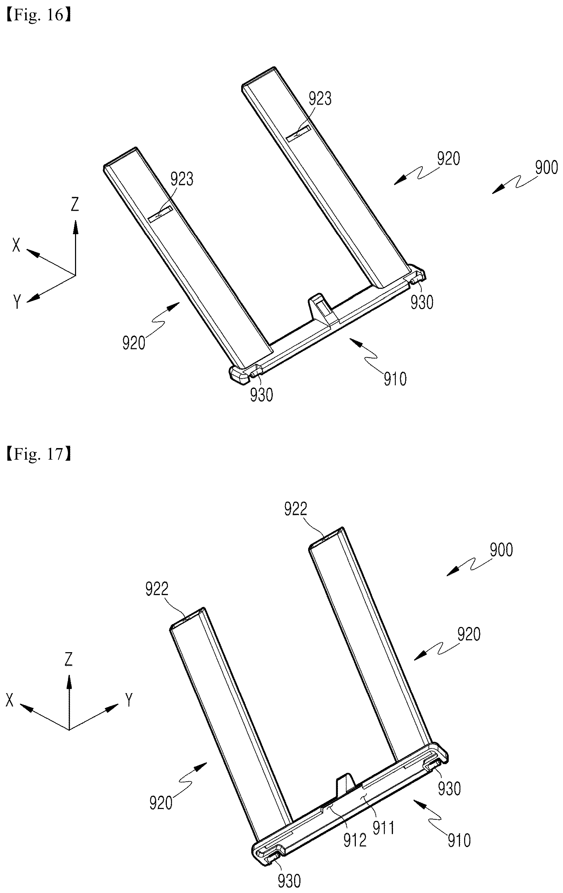

FIGS. 16 and 17 are views illustrating the shoe holding device 900 of FIG. 15 as seen from different directions, respectively.

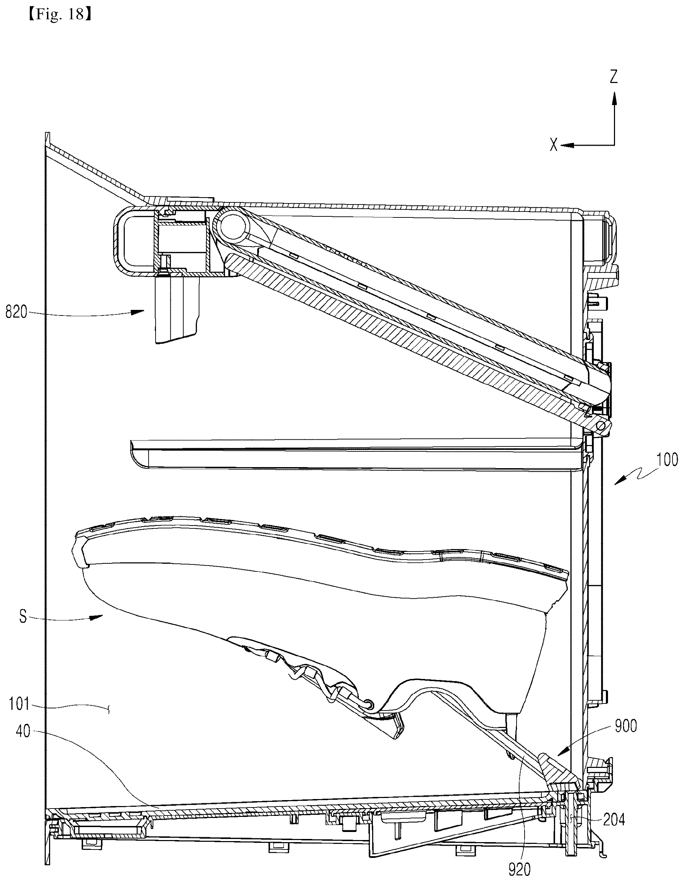

FIG. 18 is a cross-sectional view illustrating the shoe care device 1 of FIG. 15.

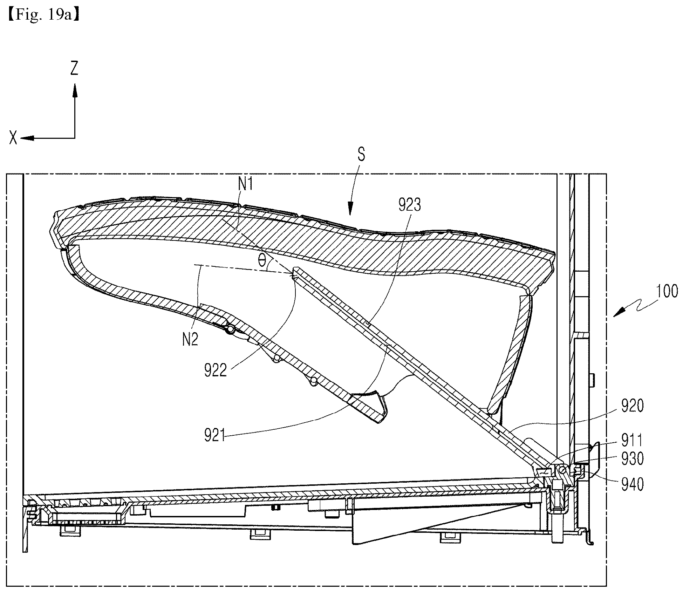

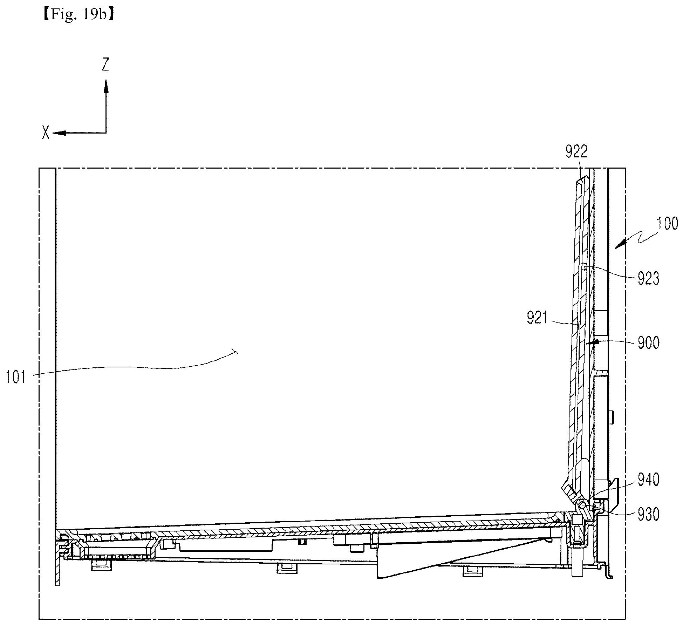

FIG. 19A is a cross-sectional view illustrating the shoe care device 1 of FIG. 15, and FIG. 19B is a cross-sectional view illustrating the shoe holding device 900 of FIG. 19A in the rotated state.

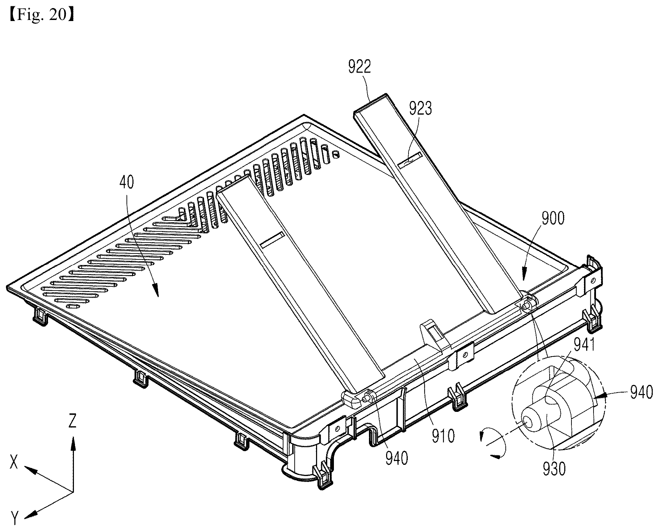

FIG. 20 is a view illustrating a partial configuration of the shoe care device 1 according to an embodiment of the present disclosure.

The shoe holding device 900 according to an embodiment of the present disclosure is configured to hold a shoe S inside the shoe care device 1. The shoe holding device 900 supports the shoe S such that the shoe S is supported in the state of being spaced apart from the inner bottom surface of the inner cabinet 100.

When the shoe S is spaced apart from the inner bottom surface of the inner cabinet 100, air can be effectively circulated to all parts of the inside and outside of the shoes, and shoe care can be achieved more effectively.

The shoe holding device 900 is detachably coupled to the inner cabinet 100.

When the use of the shoe holding device 900 is not necessary, the shoe holding device 900 may be separated from the inner cabinet 100, and in this state, a user may use the shoe care device 1 by placing a shoe S inside the inner cabinet 100.

The shoe holding device 900 includes a holding base 910 and a holding pipe 920.

When the shoe holding device 900 includes the holding base 910 and the holding pipe 920 and does not include a holding shelf 950 which will be described later, the shoe holding device 900 and a main shelf 40 may be mounted and used together inside the inner cabinet 100. At this time, except for the portion to which the shoe holding device 900 is coupled, the upper surface of the main shelf 40 may provide most of the bottom surface of the inner cabinet 100.

The holding base 910 and the holding pipe 920 are fixed to each other. The holding base 910 and the holding pipe 920 may be integrated with each other.

The holding base 910 is detachably coupled to the inner cabinet 100.

The holding base 910 is configured to shield the steam inlet 204. That is, when the shoe holding device 900 is mounted inside the inner cabinet 100, the holding base 910 shields the steam inlet 204.

The holding base 910 is located on the upper side of the steam inlet 204 and configured to shield the steam inlet 204.

The holding base 910 is provided with a first channel 911 therein. The first channel 911 provides a space inside the holding base 910 and provides a passage through which fluid moves.

The steam inlet 204 is in communication with the first channel 911, and the steam introduced through the steam inlet 204 moves to the first channel 911.

The holding pipe 920 extend upward from the holding base 910. In the shoe holding device 900 according to an embodiment of the present disclosure, steam may be moved through the inside (second channel 921) of the holding pipe 920. Since steam tends to rise upward, the holding pipe 920 extends generally upward from the holding base 910 (or extends to be inclined upward), thereby passing through the inside (second channel 921) of the holding pipe 920, whereby natural movement of steam can be achieved, and steam can be supplied smoothly to the shoe S.

The holding pipe 920 may be made to have a size and shape that can enter the inside of the shoe S.

The holding pipe 920 has a generally tubular shape. The holding pipe 920 may be made in the form of a generally thin and long pipe.

In cross section, the holding pipe 920 may have a horizontal size (width) larger than the vertical size (thickness). In addition, the holding pipe 920 has a generally constant cross section along the longitudinal direction thereof. Accordingly, the shoe S can be relatively stably supported by the holding pipe 920, when the holding pipe 920 enters the inside of the shoe S.

In the cross section of the holding pipe 920, the width of the holding pipe 920 may be 2 to 7 cm. The width of the holding pipe 920 may be 3 to 5 cm.

The holding pipe 920 may have a length sufficient to allow the shoes S held thereon to be spaced apart from the bottom of the inner cabinet 100 (e.g., the upper surface of the main shelf 40). The length of the holding pipe 920 may vary depending on the size of the shoe to be managed. The length of the holding pipe 920 may be 15 to 40 cm. The length of the holding pipe 920 may be 20 to 30 cm.

Since the holding pipe 920 extends upward from the holding base 910, the shoe S held on the holding pipe 920 is generally in an overturned state.

The holding pipe 920 include therein a second channel 921 defining a space along the longitudinal direction thereof. The second channel 921 communicates with the first channel 911. The second channel 921 may be configured to be constant along the longitudinal direction of the holding pipe 920.

A first pipe outlet 922 is provided at an end of the holding pipe 920 and communicates with a second channel 921.

In an embodiment, the opening direction N2 of the first pipe outlet 922 may be the same as the longitudinal direction N1 of the second channel 921.

In another embodiment, the opening direction N2 of the first pipe outlet 922 may bent downward at a predetermined angle θ from the longitudinal direction N1 of the second channel 921. At this time, the angle θ between the longitudinal direction N1 (the longitudinal direction of the holding pipe 920) of the second channel 921 and the opening direction N2 of the first pipe outlet 922 may be 10 to 50°. With this configuration, it is possible to prevent the first pipe outlet 922 from being covered by the shoe S while the shoe S is held on the holding pipe 920, and smooth discharge of air and steam through the first pipe outlet 922 can be achieved.

The air and/or steam in the first channel 911 may be discharged to the outside of the holding pipe 920 through the first pipe outlet 922 after moving to the second channel 921, and in the state in which the shoe S is held on the holding pipe 920, air and/or steam may be sprayed into the shoe S through the first pipe outlet 922.

Since the first pipe outlet 922 is provided at the end of the holding pipe 920 and the second channel 921, steam and/or air moving through the second channel 921 can be smoothly discharged through the first pipe outlet 922 and unnecessary flow resistance can be prevented from occurring or increasing inside the second channel 921.

In an embodiment of the present disclosure, the holding pipe 920 may be inclined upward from the holding base 910 along the horizontal direction. The holding pipe 920 may be inclined upward from the holding base 910 along the first direction (X direction) which is the horizontal direction.

The holding base 910 may be provided along the second direction (Y direction), which is a horizontal direction orthogonal to the first direction (X direction). At this time, the longitudinal direction of the holding base 910 is parallel to the second direction (Y).

When the first direction (X direction) is parallel to the front-rear direction and the second direction (Y direction) is parallel the left-right direction, the holding base 910 may be provided along the left-right direction, but the holding pipe 920 may be provided along the front-rear direction and inclined upward toward the front.

The length of the holding base 910 may vary depending on the shoes to be managed. The length of the holding base 910 may be 10 to 30 cm. The length of the holding base 910 may be about 20 cm.

The shoe holding device 900 according to an embodiment of the present disclosure may be provided with a pair of holding pipes 920 to be spaced apart from each other in the second direction (Y direction). The pair of holding pipes 920 may be made to be identical to each other. The pair of holding pipes 920 may be arranged to be horizontally symmetrical.

In the shoe holding device 900, the pair of holding pipes 920 may be provided adjacent to both ends of the holding base 910, respectively.

When managing a pair of shoes, the shoe S may be held on the holding pipes 920, respectively.

The holding pipes 920 may each include a second pipe outlet 923.

The second pipe outlet 923 is a hole provided in the holding pipe 920 so that the second channel 921 of the holding pipe 920 communicates with the outside.

When the first pipe outlet 922 is a hole provided at the end of the holding pipe 920, the second pipe outlet 923 is a hole provided at a point spaced apart from the end of the holding pipe 920.

Some of the air and/or steam moving through the second channel 921 may be discharged through the first pipe outlet 922 and some of the air and/or steam may be discharged through the second pipe outlet 923.

The second pipe outlet 923 may be provided in the holding pipe 920 at a point adjacent to the first pipe outlet 922. The second pipe outlet 923 is provided in the holding pipe 920 at a point closer to the first pipe outlet 922 than the holding base 910.

With reference to the longitudinal direction of the holding pipe 920, the length between the holding base 910 and the second pipe outlet 923 may be 1.5 to 4 times the length between the first pipe outlet 922 and the second pipe outlet 923. With reference to the longitudinal direction of the holding pipe 920, the length between the holding base 910 and the second pipe outlet 923 may be twice the length between the first pipe outlet 922 and the second pipe outlet 923.

With this configuration, both the first pipe outlet 922 and the second pipe outlet 923 can be located inside the shoe S while the shoe S is held on the holding pipe 920, and the air and/steam sprayed through the first pipe outlet 922 and the second pipe outlet 923 can be supplied into the shoe S.

As described above, the holding pipe 920 may be inclined upward in the first direction (X direction), and at this time, the second pipe outlet 923 may be provided in the upper surface of the holding pipe 920. Accordingly, some of the air and/or steam moving along the second channel 921 may be discharged generally in the first direction (X direction) (to the front space inside the shoe) through the first pipe outlet 922, and some of the air and/or steam may be discharged through the second pipe outlet 923 in a direction generally opposite to the first direction (X direction) or in an upwardly inclined direction opposite to the first direction X (to the rear space inside the shoe).

Accordingly, air and/or steam can be quickly supplied to the entire inner space of the shoe S.

When the shoe S is held on the holding pipe 920, the end of the holding pipe 920 comes into contact with the inner surface or insole portion of the shoe. At this time, depending on the opening direction of the first pipe outlet 922, part or all of the first pipe outlet 922 may be covered or spray of fluid through the first pipe outlet 922 may be hindered.

In the holding pipe 920 according to an embodiment of the present disclosure, since the second pipe outlet 923 is provided in addition to the first pipe outlet 922, even if part or all of the first pipe outlet 922 is covered by the insole of the shoe or the like, air and/or steam can be smoothly discharged through the second pipe outlet 923, and can be smoothly sprayed into the inner space of the shoe.

However, as described above, in an embodiment of the present disclosure, since the opening direction of the first pipe outlet 922 is bent downward in the longitudinal direction of the first channel 911 (the longitudinal direction of the holding pipe 920) or provided in a curved direction, the first pipe outlet 922 can be effectively prevented from being covered by the shoe, and smooth discharge of air and steam through the first pipe outlet 922 can be achieved.

The holding base 910 according to an embodiment of the present disclosure may include a base communication hole 912.

The base communication hole 912 is a hole provided in the holding base 910 and allows the inside (first channel 911) and the outside of the holding base 910 to communicate with each other.

When the holding base 910 has an open lower side, the base communication hole 912 may be provided in the form of a hole penetrating one side wall of the holding base 910.

When the holding base 910 is located inside the inner cabinet 100 adjacent to one side wall of the inner cabinet 100, the base communication hole 912 may be provided in the holding base 910 on the opposite side of the side wall of the inner cabinet 100. When the holding base 910 is located at the rear side in the inner cabinet 100 in the first direction (X direction), the base communication hole 912 may be provided at the front side in the holding base 910 in the first direction (X direction). That is, the base communication hole 912 may be provided in the holding base 910 at the side facing the inside of the inner cabinet 100.

By providing the base communication hole 912, even in the state in which the holding base 910 shields the steam inlet 204, steam and/or air in the first channel 911 in the holding base 910 can move directly into the inner cabinet 100 through the base communication hole 912.

By providing the base communication hole 912 in the holding base 910, excessive pressure increase can be prevented in the first channel 911, and some of the steam and/or air can also be directly moved into the inner cabinet 100 and supplied to the outer surfaces of the shoes through the base communication hole 912.

The shoe holding device 900 according to an embodiment of the present disclosure may include a holding rotation shaft 930.

The holding base 910 may be coupled to the inner cabinet 100 to be rotatable about the holding rotation shaft 930. The shoe holding device 900 may be coupled to the inner cabinet 100 to be rotatable about the holding rotation shaft 930.

The holding rotation shaft 930 may be parallel to the longitudinal direction of the holding base 910. The holding rotation shaft 930 may be parallel to the second direction (Y direction).

A pair of holding rotation shafts 930 may be provided.

For coupling with the holding rotation shaft 930 of the shoe holding device 900, a shaft holder 940 may be provided inside the inner cabinet 100 of the shoe care device 1.

The shaft holder 940 is provided inside the inner cabinet 100. The shaft holder 940 may be configured integrally with the inner cabinet 100, or may be configured separately from the inner cabinet 100 and then fixedly coupled to the inner cabinet 100.

The shaft holder 940 may be provided to protrude upward from the rear bottom of the inner cabinet 100. A shaft hole 941, which is a hole penetrating the shaft holder 940, is provided in the center of the shaft holder 940. The holding rotation shaft 930 may be inserted into the shaft hole 941, so that the shoe holding device 900 and the shaft holder 940 can be coupled to each other.

When the holding rotation shaft 930 is parallel to the second direction (Y direction), the penetrating direction of the shaft hole 941 is also parallel to the second direction (Y direction).

When a pair of holding rotation shafts 930 are provided, a pair of shaft holders 940 are also provided. The pair of shaft holders 940 are provided on opposite sides of the steam inlet 204, respectively.

The pair of holding rotation shafts 930 may protrude from the holding base 910 along the second direction (Y direction).

The pair of holding rotation shafts 930 protrude from the holding base 910 in the same direction. In an embodiment, the pair of holding rotation shafts 930 may both protrude from the holding base 910 in the second direction (Y direction). In another embodiment, the pair of holding rotation shafts 930 may both protrude from the holding base 910 in a direction opposite to the second direction (Y direction).

Since the pair of holding rotation shafts 930 protrude from the holding base 910 in the same direction, a user may easily separate the shoe holding device 900 from the shaft holders 940 by moving the holding base 910 in the opposite direction to the protruding direction of the holding rotation axis 930.

For example, when the pair of holding rotation shafts 930 both protrude from the holding base 910 in the second direction (Y direction), the user may move the holding base 910 from the shaft holders 940 in the opposite direction to the second direction (Y direction). As a result, the pair of holding rotation shafts 930 can be broken away from the shaft holes 941 in the shaft holders 940 and can be separated from the shaft holders 940.

In an embodiment of the present disclosure, the holding rotation shafts 930 may be provided at the rear side of the holding base 910 with reference to the first direction (X direction).

With reference to the first direction (X direction), the holding pipes 920 extend to be inclined upward from the holding base 910 forward, and the holding rotation shafts 930 are provided at the rear side of the holding base 910. As a result, in the state in which no separate external force is applied, the shoe holding device 900 is maintained in a form in which the holder base 910 shields the steam inlet 204.

At this time, when an external force is applied to the shoe holding device 900 to rotate the shoe holding device 900 around the holding rotation shafts 930, the holding pipes 920 approach a side wall (the inner rear plate 110) of the inner cabinet 100, and the holding base 910 exposes the steam inlet 204.

In some embodiments, when the shoe holding device 900 is rotated, the ends of the holding pipes 920 may be brought into contact with the side wall (the inner rear plate 110) of the inner cabinet 100.

Since the shoe holding device 900 is configured to be rotatable inside the inner cabinet 100, when it is not necessary to use the shoe holding device 900, the holding pipes 920 may be disposed to be in contact with or close to the side wall (the inner rear plate 110) of the inner cabinet 100, and the inner space (the accommodation space 101) of the inner cabinet 100 may be effectively utilized.

In addition, when it is necessary to use the shoe holding device 900, when the holding pipes 920 are inserted into shoes S to hold the shoes S, the holding rotation shafts 930 are stably supported by the shaft holders 940 even when the weight of the shoes S is applied to the holding pipes 920.

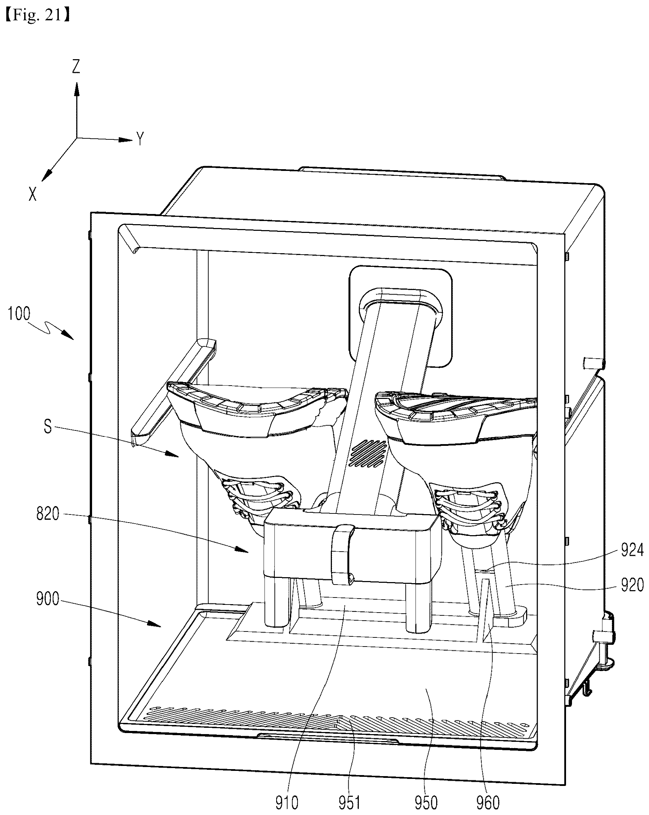

FIG. 21 is a view illustrating a portion of the shoe care device 1 of the present disclosure in the state in which shoes S are held on the shoe holding device 900 inside the inner cabinet 100.

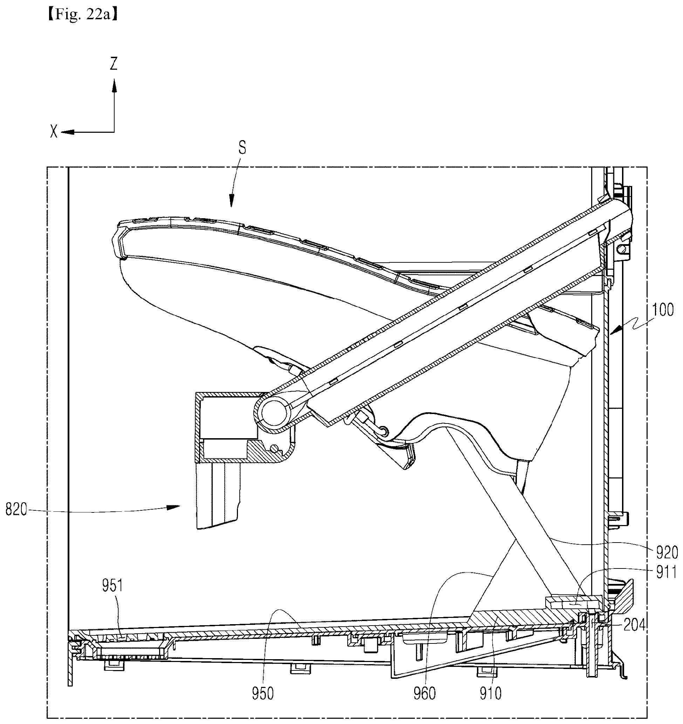

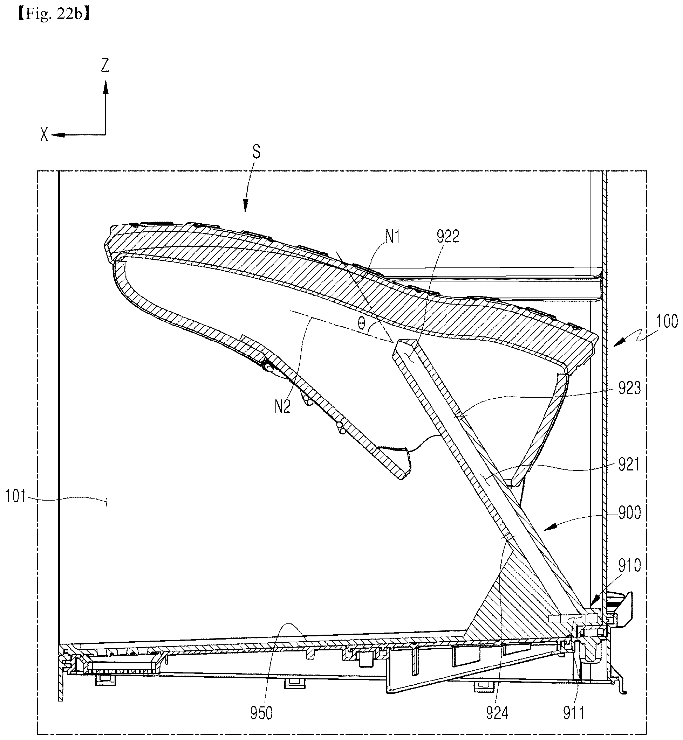

FIGS. 22A and 22B are cross-sectional views each illustrating the shoe care device 1 of FIG. 21.

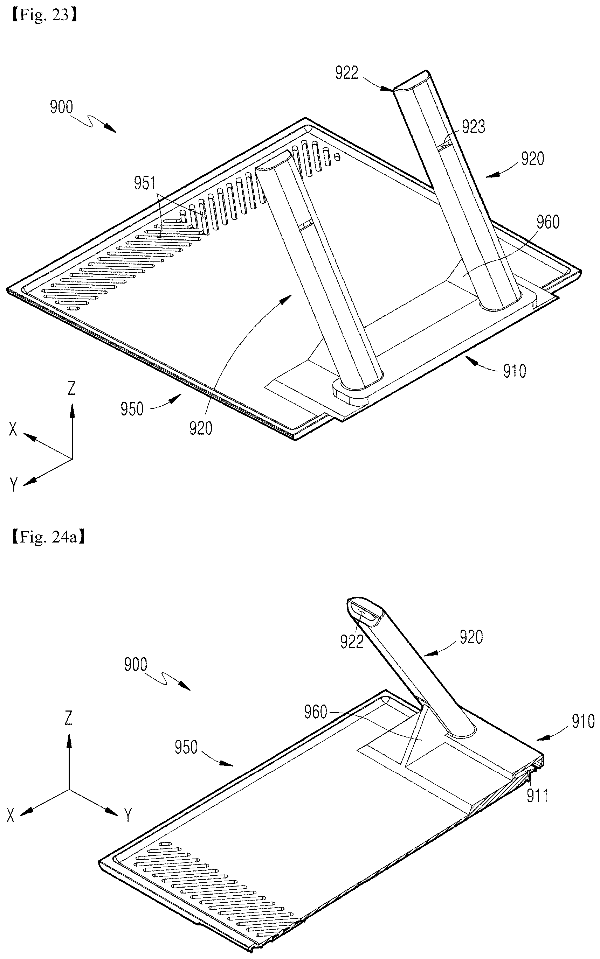

FIG. 23 is a view illustrating the shoe holding device 900 illustrated in FIG. 21.

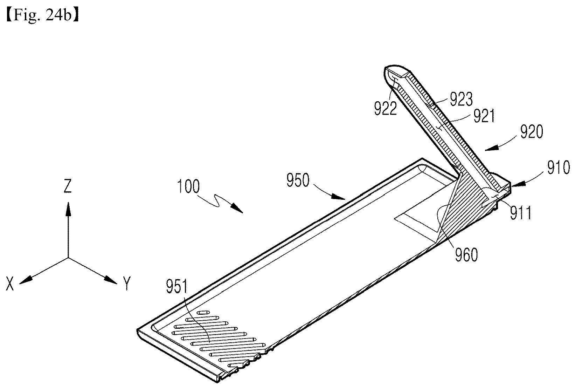

FIGS. 24A and 24B are cross-sectional perspective views illustrating the shoe holding device 900 illustrated in FIG. 23 when viewed in different directions, respectively.

Each holding pipe 920 may include a third pipe outlet 924.

The third pipe outlet 924 is a hole provided in the holding pipe 920 so that the inside (the second channel 921) of the holding pipe 920 communicates with the outside.

When the first pipe outlet 922 is a hole provided at the end of the holding pipe 920, the third pipe outlet 924 is a hole provided at a point sufficiently spaced apart from the end of the holding pipe 920.

The third pipe outlet 924 may be provided in the holding pipe 920 at a point spaced apart from the holding base 910.

Some of the air and/or steam moving through the second channel 921 may be discharged through the first pipe outlet 922, and some of the air and/or steam may be discharged through the third pipe outlet 924.

The third pipe outlet 924 may be provided in the holding pipe 920 at a point adjacent to the holding base 910. The third pipe outlet 924 is provided in the holding pipe 920 at a point closer to the holding base 910 than the first pipe outlet 922.

With reference to the longitudinal direction of the holding pipe 920, the length between the first pipe outlet 922 and the third pipe outlet 924 is 1.5 to 4 times the length between the holding base 910 and the third pipe outlet 924. With reference to the longitudinal direction of the holding pipe 920, the length between the first pipe outlet 922 and the third pipe outlet 924 is two or more times the length between the holding base 910 and the third pipe outlet 924.

With this configuration, while a shoe S is held on the holding pipe 920, when the first pipe outlet 922 (and the second pipe outlet 923) is located inside the shoe S, the third pipe outlet 924 may be located outside the shoe S. The air and/or steam sprayed through the first pipe outlet 922 (and the second pipe outlet 923) may be supplied to the inside of the shoe S, and the air and/or steam sprayed through the third pipe outlet 924 may be supplied to the outside of the shoe S.

As described above, the holding pipe 920 may be inclined upward, and in this case, the third pipe outlet 924 may be provided in the lower surface of the holding pipe 920. Accordingly, some of the air and/or steam moving along the second channel 921 may be discharged into the shoe through the first pipe outlet 922 (and the second pipe outlet 923), and some of the air and/or steam may be discharged toward the center of the inner cabinet 100 through the third pipe outlet 924.

Therefore, the air and/or steam is quickly supplied to the inner space of the shoe, and some of the air and/or steam is supplied to the outside of the shoe, so that proper shoe care can be achieved.

The shoe holding device 900 may further include a holding shelf 950.

The holding shelf 950 is flat along the horizontal direction so as to be placed on the bottom of the inner cabinet 100, and is fixed to the holding base 910.

When the shoe holding device 900 includes the holding shelf 950 and is seated inside the inner cabinet 100, the use of the main shelf 40 is excluded. The holding shelf 950 may replace the main shelf 40.

With reference to the holding base 910 in a plan view, the direction in which the holding shelf 950 is located is the same as the direction in which the holding pipes 920 are located. That is, in the plan view, when the holding pipes 920 extend in the first direction (X direction) with reference to the holding base 910, the holding shelf 950 also extends in the first direction (X direction).

The holding shelf 950 may be configured such that shoes S are seated on its upper surface.

The holding shelf 950 may be provided in the form of a plate having a predetermined area, or may be provided in the form of a grill in which a plurality of bars are spaced apart from each other.

The holding shelf 950 may be provided in the form of a generally flat plate and placed on the bottom of the inner cabinet 100.

The holding shelf 950 is detachable from the inner cabinet 100, and when the holding shelf 950 is pulled out from the inner cabinet 100, the bottom surface of the inner cabinet 100 is exposed.

The holding shelf 950 may have a square shape in a plan view. The holding shelf 950 may have a size corresponding to the bottom of the accommodation space 101 of the inner cabinet 100. That is, when the holding shelf 950 is placed inside the inner cabinet 100, the holding shelf 950 may form all or most of the bottom of the accommodation space 101 of the inner cabinet 100.

The holding shelf 950 may be provided with a plurality of holding shelf holes 951 vertically penetrating the holding shelf 950 to allow air to move. The holding shelf holes 951 may be provided in the front portion of the holding shelf 950 with reference to the first direction (X direction).

The holding shelf 950 may be the same as or similar to the main shelf 40.

With reference to the first direction (X direction), the length of the holding shelf 950 may be greater than the length of the holding pipes 920. Accordingly, in the state in which the shoes S are held on the holding pipes 920, the centers of gravity of the shoes S and the holding pipes 920 can be located within the area of the holding shelf 950 in a plan view, and the shoes can be held stably.

The shoe holding device 900 may further include reinforcement ribs 960.

The reinforcement ribs 960 are provided to directly interconnect the holding pipes 920 and the holding shelf 950.

The reinforcement ribs 960 may be fixedly coupled to the holding pipes 920 and the holding shelf 950. The reinforcement ribs 960 may be configured integrally with the holding pipes 920 and the holding shelf 950.

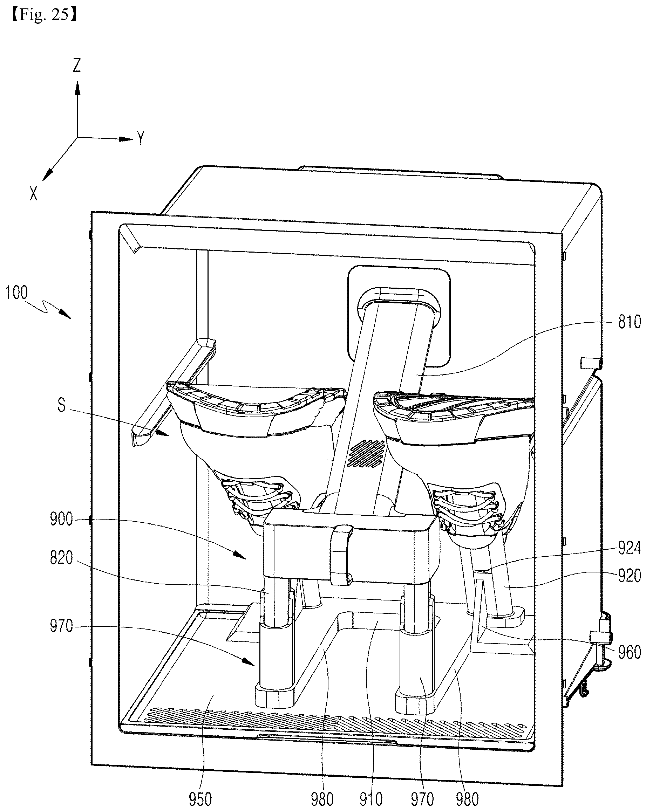

FIG. 25 is a view illustrating a portion of the shoe care device 1 of the present disclosure in the state in which shoes S are held on the shoe holding device 900 inside the inner cabinet 100.

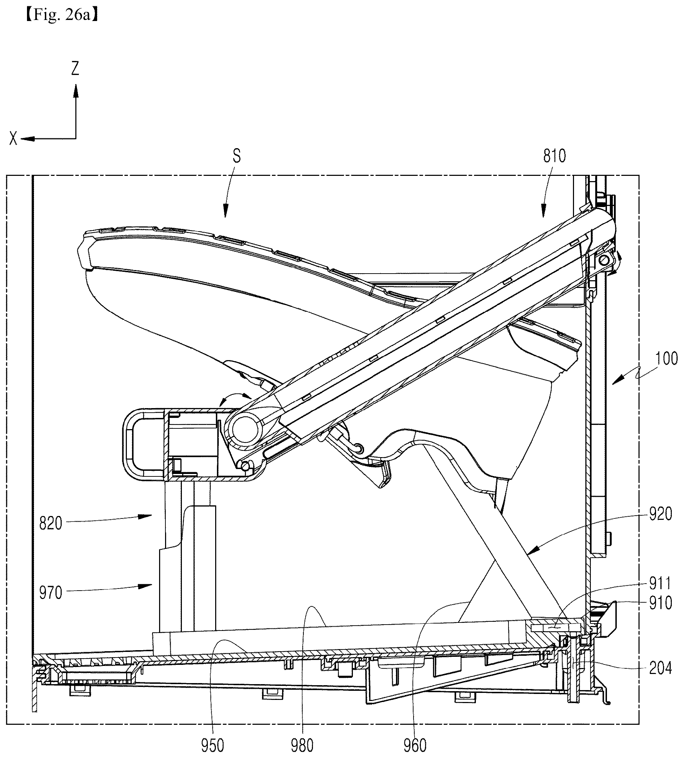

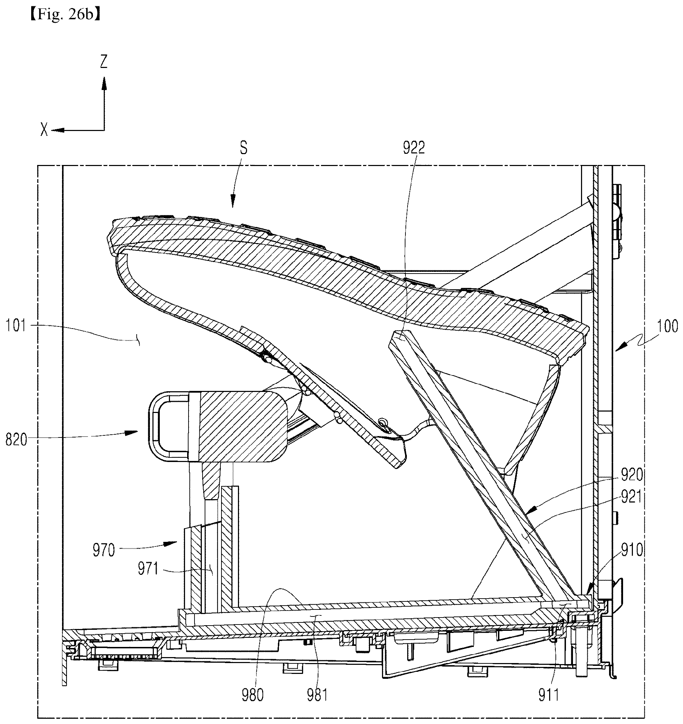

FIGS. 26A and 26B are cross-sectional views each illustrating the shoe care device 1 of FIG. 25.

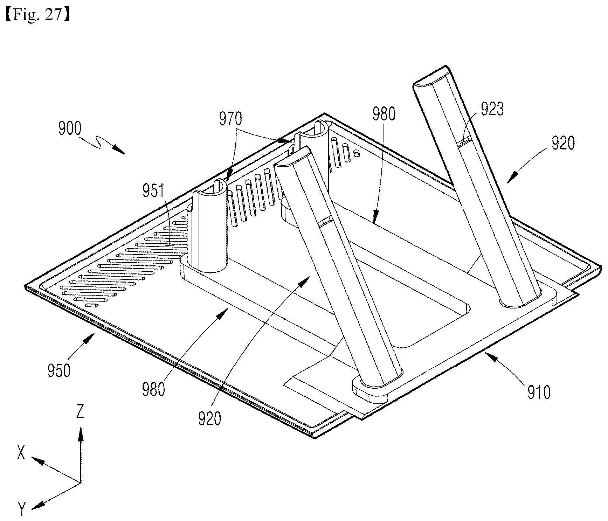

FIG. 27 is a view illustrating the shoe holding device 900 illustrated in FIG. 25.

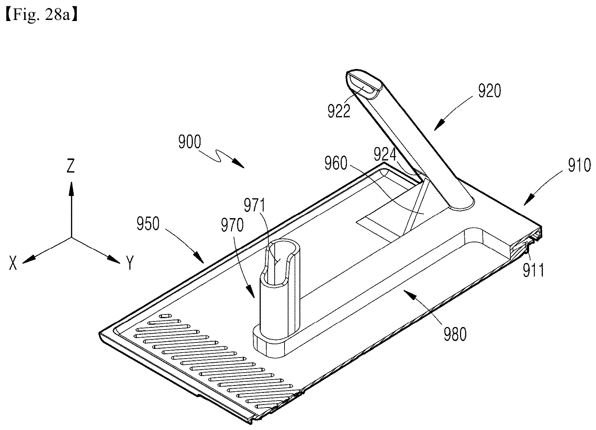

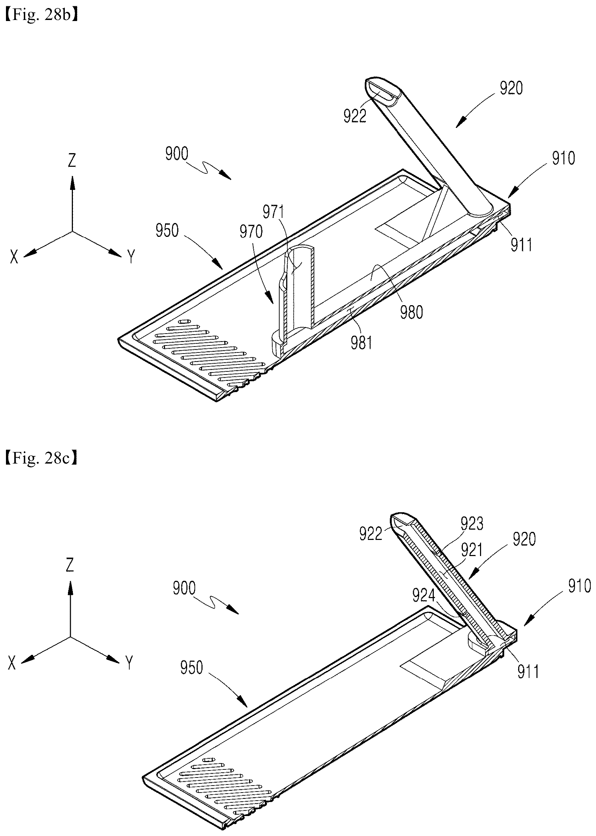

FIGS. 28A to 28C are cross-sectional perspective views illustrating the shoe holding device 900 illustrated in FIG. 27 when viewed in different directions, respectively.

The shoe holding device 900 may further include nozzle connection parts 970 and channel connection parts 980.

The nozzle connection parts 970 protrude upward from the upper surface of the holding shelf 950 and are connected to the nozzles 820, respectively.

The nozzle connection parts 970 are each provided in the form of a generally vertical pipe. The inner diameter of the nozzle connection parts 970 may be the same or almost the same as the outer diameter of the nozzles 820. The nozzles 820 may be coupled to the nozzle connection parts 970 in the form of being fitted into the nozzle connection parts 970, respectively.

Inside the nozzle connection parts 970, third channels 971 are provided as passages through which air can move.

In the state in which the nozzles 820 and the nozzle connection parts 970 are coupled, the air discharged from the nozzles 820 flows into the third channels 971 in the nozzle connection parts 970.

The nozzle connection parts 970 may be provided in a pair and may be spaced apart from each other.

The channel connection parts 980 are provided on the upper surface of the holding shelf 950. The channel connection parts 980 may be provided in parallel to the direction of the upper surface of the holding shelf 950. The channel connection parts 980 may be provided along the first direction (X direction).

The channel connection parts 980 are connected to the nozzle connection parts 970, respectively. When the nozzle connection parts 970 are provided in a pair, the channel connection parts 980 may be provided in a pair and may be spaced apart from each other.

The channel connection parts 980 are provided in the form of a generally horizontal pipe. Inside the channel connection parts 980, fourth channels 981, which are passages through which air moves, are provided.

The fourth channel 981 are in communication with the second channels 921 and the third channels 971. That is, air flowing into the third channels 971 of the nozzle connection parts 970 may move to the second channels 921 through the fourth channels 981 in the channel connection parts 980.

In an embodiment, the fourth channels 981 in the channel connection parts 980 may be directly connected to the second channels 921.

In another embodiment, the fourth channels 981 in the channel connection parts 980 may be connected to the second channels 921 through the first channels 911. That is, the channel connection parts 980 are connected to the holding base 910 so that the fourth channels 981 are directly connected to the first channels 911.

As described above, the shoe care device 1 according to an embodiment of the present disclosure may include a heating part 320 and a dehumidifying part 330.

The heating part 320 is provided under the bottom of the inner cabinet 100 to which the shoe holding device 900 is coupled, and is configured to heat the air introduced from the inner cabinet 100.

A plurality of holding shelf holes 951, which are through holes, are provided in the holding shelf 950 of the shoe holding device 900, and the air inside the inner cabinet 100 are movable toward the heating part 320 through the holding shelf holes 951.

The dehumidifying part 330 is configured to dehumidify the air introduced from the inner cabinet 100.

The air inside the inner cabinet 100 may sequentially pass through the heating part 320 and the dehumidifying part 330, and the air passing through the dehumidifying part 330 may be supplied again into the inner cabinet through the nozzles 820.

As described above, the inner cabinet 100 includes a main opening 140 opening on one side, and the shoe care device 1 includes a door 30 configured to open/close the main opening 140. The steam inlet 204 through which steam is supplied into the inner cabinet 100 may be provided on the rear bottom of the inner cabinet 100 opposite to the door 30.

As described above, a heating part 320 is provided under the bottom of the inner cabinet 100 to which the shoe holding device 900 is coupled, and the heating part 320 heats the air introduced from the inner cabinet 100. The temperature and humidity of the air used for shoe care can be controlled by operating the heating part 320. In this case, when the shoes are held by the shoe holding device 900, the shoes S can be spaced apart from the heating part 320 so that the shoes S can be prevented from being damaged by heat from the heating part 320.

For example, in the case of some shoes, the soles may be bonded by adhesive. When the heating part 320 is heated while the shoes S are placed on the bottom of the inner cabinet 100, the high temperature heat of the heating part 320 may be transferred to the soles of the shoes and melt the adhesive, which may cause damage to the shoes.

When using the shoe holding device 900 according to an embodiment of the present disclosure, damage to the shoes can be prevented by separating the shoes away from the bottom of the inner cabinet 100, steam and/or air required for shoe care can be intensively supplied to the shoes, and effective shoe care can be achieved.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### [Industrial Applicability]

A shoe holding device and a shoe care device including the same according to an embodiment of the present invention have significant industrial applicability in that they enable simultaneous shoe mounting and steam supply, with the steam being delivered to the shoes in a concentrated and effective manner.

## Claims

1. A shoe holding device configured to hold a shoe in a shoe care device having a steam inlet configured to supply steam into an inner cabinet and a nozzle configured to supply air into the inner cabinet, the shoe holding device comprising:
a holding base detachably coupled to the inner cabinet and configured to shield the steam inlet, the holding base comprising a first channel provided therein to communicate with the steam inlet; and
a holding pipe extending upward from the holding base and configured to enter the shoe, the holding pipe comprising a second channel provided therein to communicate with the first channel and a first pipe outlet provided at an end thereof to communicate with the second channel.

2. The shoe holding device of claim 1, wherein the holding pipe is provided to be inclined upward from the holding base along a first direction which is a horizontal direction, and
wherein the holding base is provided along a second direction, which is a horizontal direction orthogonal to the first direction.

3. The shoe holding device of claim 2, wherein a pair of holding pipes are provided to be spaced apart from each other in the second direction.

4. The shoe holding device of claim 1, wherein the holding pipe comprises a second pipe outlet as a hole provided at a point closer to the first pipe outlet than the holding base and communicating with the second channel.

5. The shoe holding device of claim 1, wherein the holding base comprises a base communication hole, which is a hole penetrating the holding base, to allow the first channel to communicate with an inside of the inner cabinet.

6. The shoe holding device of claim 1, wherein the holding pipe comprises a third pipe outlet, which is a hole provided at a point closer to the holding base than the first pipe outlet, to allow the second channel to communicate with an inside of the inner cabinet.

7. The shoe holding device of claim 2, wherein the holding pipe comprises a second pipe outlet as a hole provided at a point closer to the first pipe outlet than the holding base and communicating with the second channel, and
wherein the second pipe outlet is provided in an upper surface of the holding pipe.

8. The shoe holding device of claim 2, wherein the holding base is coupled to the inner cabinet to be rotatable about a holding rotation shaft, and
wherein the holding rotation shaft is parallel to the second direction.

9. The shoe holding device of claim 8, wherein a pair of holding rotation shafts are provided, and
wherein the pair of holding rotation shafts protrude from the holding base in a same direction.

10. The shoe holding device of claim 8, wherein the holding rotation shaft is provided at a rear side of the holding base with reference to the first direction, and
wherein, when the shoe holding device rotates to one side about the holding rotation shaft, the holding pipe approaches a side wall of the inner cabinet and the holding base exposes the steam inlet.

11. The shoe holding device of claim 1, further comprising:
a holding shelf that is flat along the horizontal direction to be placed on a bottom of the inner cabinet and is fixed to the holding base.

12. The shoe holding device of claim 2, further comprising:
a holding shelf that is flat along the horizontal direction to be placed on a bottom of the inner cabinet and is fixed to the holding base,
wherein, with reference to the first direction, the holding shelf has a length greater than a length of the holding pipe.

13. The shoe holding device of claim 12, further comprising:
a reinforcement rib directly interconnecting the holding pipe and the holding shelf.

14. The shoe holding device of claim 1, further comprising:
a holding shelf that is flat along the horizontal direction to be placed on a bottom of the inner cabinet and is fixed to the holding base;
a nozzle connection part protruding upward from an upper surface of the holding shelf to be connected to the nozzle, the nozzle connection part comprising therein a third channel into which air discharged from the nozzle is introduced; and
a channel connection part provided on the upper surface of the holding shelf, the channel connection part comprising a fourth channel provided therein to communicate with the second channel and the third channel.

15. The shoe holding device of claim 14, wherein the fourth channel is connected to the second channel via the first channel.

16. A shoe care device comprising:
a steam inlet configured to supply steam into an inner cabinet;
a nozzle configured to supply air into the inner cabinet;
a shoe holding device configured to hold a shoe; and
a heating part provided below a bottom of the inner cabinet to which the shoe holding device is coupled, and configured to heat air introduced from the inner cabinet,
wherein the shoe holding device comprises:
a holding base detachably coupled to the inner cabinet and configured to shield the steam inlet, the holding base comprising a first channel provided therein to communicate with the steam inlet; and
a holding pipe extending upward from the holding base and configured to enter the shoe, the holding pipe comprising a second channel provided therein to communicate with the first channel and a first pipe outlet provided at an end thereof to communicate with the second channel.

17. The shoe care device of claim 16, further comprising:
a dehumidifying part configured to dehumidify the air introduced from the inner cabinet,
wherein the air passing through the dehumidifying part is supplied into the inner cabinet through a nozzle.

18. The shoe care device of claim 16, wherein the inner cabinet comprises a main opening that opens on one side, wherein the shoe care device further comprises a door configured to open/close the main opening, and wherein the steam inlet configured to supply steam into the inner cabinet is provided at a rear bottom of the inner cabinet opposite to the door.

19. A shoe care device comprising:
a steam inlet configured to supply steam into an inner cabinet;
a nozzle configured to supply air into the inner cabinet;
a shoe holding device configured to hold a shoe; and
a heating part provided below a bottom of the inner cabinet to which the shoe holding device is coupled, and configured to heat air introduced from the inner cabinet,
wherein the shoe holding device comprises:
a holding base detachably coupled to the inner cabinet and configured to shield the steam inlet, the holding base comprising a first channel provided therein to communicate with the steam inlet;
a holding pipe extending upward from the holding base and configured to enter the shoe, the holding pipe comprising a second channel provided therein to communicate with the first channel and a first pipe outlet provided at an end thereof to communicate with the second channel; and
a holding shelf that is flat along the horizontal direction to be placed on a bottom of the inner cabinet and is fixed to the holding base,
wherein the holding shelf has a plurality of holding shelf holes provided through the holding shelf to allow air inside the inner cabinet to move toward the heating part.
